# EUROPEAN PATENT SPECIFICATION

(11) **EP 1 027 604 B1**
(45) Date of publication and mention of the grant of the patent: **04.08.2004**
(21) Application number: 98956381.2
(22) Date of filing: 30.10.1998
(51) Int. Cl.: G01N 33/543, G01N 33/53, G01N 33/58, C12Q 1/68

(54) **EXTENDED DYNAMIC RANGE ASSAYS**
VERSUCHSANORDNUNG MIT ERWEITERTER BANDBREITE
ESSAIS A PLAGE DYNAMIQUE ETENDUE

(30) Priority: 31.10.1997 US 962033
(43) Date of publication of application: 16.08.2000
(73) Proprietor: Gen-Probe Incorporated, San Diego, CA 92121-4362 (US)
(72) Inventor: NELSON, Norman, C., San Diego, CA 92111 (US)
(74) Representative: Viering, Jentschura & Partner
(86) International application number: PCT/US1998/023088
(87) International publication number: WO 1999/023490

(56) References cited:
- EP-A- 0 709 466
- WO-A-89/11101
- WO-A-95/17674
- WO-A-95/17675
- US-A- 4 595 661

## Description

This invention relates to compositions and methods for detecting analytes which may be present in a wide range of possible concentrations through the use of at least two labeled binding partners or probes, and specifically relates to using labeled binding partners or probes that specifically bind mutually exclusive target regions of the same analyte, such that the use of the at least two binding partners or probes extends the dynamic range of an assay for analyte detection.

### BACKGROUND OF THE INVENTION

Assays for the detection and/or quantification of analytes exist in many different forms and formats. In many cases, the amount of the analyte in a sample is not great enough for direct detection or quantification. In such cases, a secondary molecule capable of binding to or interacting with the analyte must be used to indicate the presence or amount of analyte in a sample. Unless the secondary molecule is directly detectable, the secondary molecule must be conjugated with a label which will be detected when the secondary molecule binds the analyte. For the purposes of the present application, such secondary molecules capable of binding or interacting with analytes are referred to as "binding partners" or "probes." Examples of detectable analytes include antibodies, proteins, cell-surface receptors, cytokines, hormones, antigens, nucleic acids, metals, molecular complexes such as polymeric arrangements of proteins or other macromolecules, and the like. Likewise, binding partners or probes for detecting such analytes may be antibodies, proteins, antigens, haptens, nucleic acid probes, chelating agents, enzymes, enzyme substrates, and analogs of these.

One commonly used assay format is the enzyme-linked immuno-absorption assay (ELISA). In this assay format, the analyte is contacted with a primary antibody capable of binding at least one domain or "target region" of the analyte. After the excess antibody is washed free of the resulting analyte: antibody complex, the primary antibody is specifically bound with an enzyme-labeled secondary antibody. A chromogenic enzyme substrate is then provided and incubated under conditions favoring enzyme-mediated reaction of the substrate. The resulting colored product and its intensity after a reaction time are indications of the presence and amount, respectively, of the analyte originally present in the sample. Suitable enzymes used in ELISA are, for example, β-galactosidase, acid phosphatase, and alkaline phosphatase and suitable enzyme substrates for use with such enzymes include x-gal (5-bromo-4-chloro-3-indolyl-β-D-galacto-pyranoside) and p-nitrophenyl phosphate, although other enzymes and substrates are well known by those skilled in the art. Variations of ELISA exist; for example, the primary antibody may be linked to an enzyme, thus eliminating the secondary antibody step. Nevertheless, these assay methods include common steps of contacting an analyte with a labeled binding partner and subsequently detecting the analyte-bound label as an indication of the presence or amount of the analyte.

Frengen (US Pat. No. 5,739,042) describes a binary assay system in which two independently determinable forms of solid-supported binding partners (e.g., monoclonal antibodies) are reacted successively rather than simultaneously with analyte and labeled ligand. The analyte concentration is obtained from readings derived from these two forms by comparison to a double standard curve.

Campbell, et al. (US Pat. No. 4,496,958) describe chemiluminescent acridinium labeling compounds for use in labeling binding partners in multiple assay formats. European Patent Application No. 0709466 describes compositions and methods for simultaneously detecting and quantifying multiple nucleic acid analytes in a single sample. Other labeling compounds such as radionuclides, fluorescent, bioluminescent, phosphorescent, luminescent, chemiluminescent, or electrochemiluminescent compounds, chromophores, and dyes are known in the art and are commonly used as labeling agents in a variety of assay formats, both direct and indirect, including immunoassay and nucleic acid hybridization assays.

Assays are distinguished based on the type of analytes to be detected, the type of binding partner with which the analyte binds, and the type of label used. Assays can also be classified based on whether the method involves the immobilization of the analyte or the analyte: binding partner complex. In a common assay format known as a "heterogeneous" or biphasic assay system, a probe is allowed to bind its analyte, usually under conditions of probe excess. Either the analyte or the probe may be immobilized to a solid support, thus immobilizing the resulting probe: analyte complex. Often, the complex is formed in the liquid phase and then immobilized. After probe: analyte complexes have been immobilized, the excess uncomplexed probes are washed away. It the probes are directly labeled, the label is then detected as an indication of the presence of analyte. Alternatively, probe: analyte complexes may be separated from free probe, by means such as gel filtration chromatography, electrophoresis, electrofocusing, or other methods that separate based on the size or charge of the probe: analyte complex.

Other assays, termed "homogeneous" assays, take place wholly in a single phase without a step to separate probe: analyte complex from free probe. In these, usually either the probe: analyte complex, or the free probe is altered after formation of the complex to permit the separate detection of analyte in the presence of the free probe. One way of differentiating free probe from probe that is bound to analyte involves alteration or selective inactivation of the label joined to the probe rather than the free probe itself. Arnold, et al. (US Pat. No. 5,283,174) describe homogeneous methods employing a oligonucleotide probe joined to a label which is capable of selective inactivation or alteration based on whether the labeled probe is bound to its target or not. These methods may be used in a single tube without the need for washing or decanting. Another homogeneous assay uses an energy-transfer system that requires close proximity of light emitting species and a light absorbing species whose absorbance spectrum overlaps the emission spectrum of the light emitting species (Canadian Pat. No. 1185177 to Morrison et al.). This assay method uses multiple antibodies to detect an antigen having multiple binding sites: one antibody is conjugated to a chemiluminescent catalyst that emits light in the presence of suitable chemiluminescent reagents and the other antibody (the "absorber/emitter") is conjugated to a moiety that absorbs light at one wavelength and emits light at another wavelength. When the separately-labeled antibodies bind to a single antigen in close proximity, highly efficient energy transfer occurs from the chemiluminescent catalyst conjugated antibody to the absorber/emitter, producing a light signal that is related to the amount of antigen.

Assays exist which utilize aspects of both homogeneous and heterogeneous assays, termed "hybrid" assays. These methods may, for example, employ a single-phase selective alteration of the probe or the probe: analyte complex, followed by a separation step to further decrease the level of background in the assay (e.g., see US Pat. No. 5,658,737 to Nelson et al.)

Regardless of the assay format used, a number of factors exist in all assays which can limit their sensitivity and the range of possible analyte concentrations that can be accurately detected or measured. One factor is the level of background present in the assay. "Background" generally describes probe or label in the assay which is not bound specifically to analyte and which may mask positive results at low analyte levels. For example, in a heterogeneous assay, background may be provided by a small amount of probe which is not removed during the physical separation of probe: analyte complex from free probe. If the probe is labeled, this small amount of probe will be detected as a residual level of detectable signal. In a homogeneous assay, background may be provided by the inability to totally alter all free probe or probe-linked label molecules. In either case, a low level of signal, commonly between 0.001% and 10% of the total signal, more commonly between about 0.01% and 1%, is present as a "baseline" below which results cannot be relied on for accuracy. Thus, the level of background inherent in a particular assay limits the lowest amount of analyte which can be detected and/or measured.

Background in an assay plays a part in limiting the "dynamic range" of the assay. By "dynamic range" is meant a linear or predictably accurate correspondence between the level of analyte present in the assayed sample and the amount of signal obtained from the label that indicates the analyte's presence. It is readily apparent to those skilled in the art that the dynamic range of an assay cannot extend below the background level contributed by the detection of non-specific label. Thus, the higher the background, the more limited the dynamic range. Moreover, if high amounts of analyte are to be detected, correspondingly high amounts of probe must be used, leading to higher backgrounds.

Other factors may limit an assay's dynamic range. Often, a limitation is the maximum amount of signal capable of being detected or reported by the label-detection device used in the assay. Thus, if an instrument can only accurately read up to, for example, one million counts per second and the sample yields two million counts per second, the extra one million counts are not reported by the instrument, and the upper extent of the assay dynamic range is half of what is necessary to accurately quantify the sample.

Also, instruments used to detect the labeled probe: analyte complexes may have inherent electronic "noise" (sometimes also referred to as "background") and which can also contribute to limitations on the accuracy of analyte detection. Although an instrument's electronic signal-to-noise ratio may be improved, noise combines with the inherent assay background described above to further limit the ability to detect or quantify analytes in a wide range of concentrations. To overcome these limitations, practitioners of assays generally obtain multiple samples from a single source, or test serial dilutions of a sample to detect the presence of an unknown amount of analyte.

Some methods increase sensitivity of an assay without extending the dynamic range of the reaction. That is, these methods simply allow one to detect a lesser amount of analyte than previously detected in the same sort of reaction. Some methods of increasing sensitivity rely on using mixtures of binding partners. For example, Canfield et al. (US Pat. No. 4,514,505) describe an antigen detection method with enhanced sensitivity that uses mixtures of monoclonal antibodies containing at least two antibodies that can bind simultaneously to at least two different antigenic sites. The amounts of each monoclonal antibody vary, but generally, the preferred amount of each antibody relative to the amount of the other antibodies is substantially the same as the ratio of the binding constants of the antibodies to the antigen to be detected. Another assay method uses nucleic acid binding partners to test for bacterial contamination of food by using one or more *Salmonella*-specific labeled probes to bind to a single *Salmonella* chromosome (European Pat. App. No. 0114668). In this system, multiple probes are used to increase the assay sensitivity because each chromosome binds multiple labels.

There exists a need in the art for methods and compositions for detecting and/or quantifying analytes in a single assay without the need for making sample dilutions or testing duplicate samples. Preferably, such methods and compositions would involve a single addition of probes for detection of analyte concentrations which may differ over a range of several orders of magnitude in different samples. Such methods are also preferably independent of the analyte type, probe type, label type, and instrument to be used for the detection of analyte, thus allowing for modifications specific for the analyte to be detected or the compositions or devices to be used.

### SUMMARY OF THE INVENTION

One aspect of the present invention is a method of detecting an analyte over a range of concentrations in a single sample. This method includes the steps of: providing a probe reagent comprising at least two probes, wherein each probe comprises a target binding moiety and a detectable label, and wherein each probe is present in an amount capable of detecting a range of analyte concentrations that differs from the range of analyte concentrations detectable by another probe in the reagent wherein the probe reagent contains a first probe present in at least a 10-fold molar excess relative to a second probe, such that the probe reagent is capable of detecting a range of analyte concentrations that is greater than the range of analyte concentrations detectable by a single probe in the reagent; and contacting in a single vessel the probe reagent with a sample containing an analyte. Then, the method includes the steps of incubating the sample and the probe reagent under conditions favoring specific binding of the target binding moiety of each probe with a target region of the analyte, wherein the target region recognized by each probe differs from the target region recognized by another probe in the probe reagent, thereby producing a specific binding complex comprising the analyte and at least one probe; and detecting the presence of the specific binding complex by detecting a signal from a label indicating the presence of at least one probe in the specific binding complex, thereby indicating the presence of the analyte in the sample within the range of analyte concentrations detectable by the probe in the specific binding complex. In one embodiment, the detectable label of one probe in the probe reagent results in a signal that is distinguishable from a signal resulting from the detectable label of another probe in the reagent. In another embodiment, the detecting step measures signal characteristic of kinetics of a reaction. In a preferred embodiment, the providing step uses a probe reagent in which specific activity of one probe differs from specific activity of another probe in the reagent. In another preferred embodiment, the providing step uses a probe reagent wherein the detectable label of a first probe results in a signal that is distinguishable from a signal resulting from the detectable label of a second probe in the reagent, and wherein specific activity of the first probe differs from specific activity of the second probe in the reagent. Preferably, the signal resulting from the first probe is distinguishable from the signal resulting from the second probe by measuring kinetics of a reaction in the detecting step. In one embodiment, the detectable label of at least one probe is a direct label such that the detecting step measures a signal resulting from the label. In another embodiment, at least one probe has a detectable label that is a fluorescent label, a luminescent label, a radioactive label, a chromophore, an enzyme, or an enzyme substrate. Preferably, the detectable label is a chemiluminescent label. In one embodiment of the method, at least one probe of the probe reagent has a detectable label that is a ligand capable of specifically binding a signal-producing binding partner. Preferably, the detecting step detects a signal that is light emittance, light absorbance, radioactive decay, a precipitate, or a product of an enzymatic reaction. In one embodiment of the method, the contacting step uses a probe reagent wherein a first probe is present in at least a 10-fold molar excess relative to a second probe. In other embodiments, the first probe is present in at least a 100-fold molar excess, or at least a 1,000-fold molar excess, or at least a 10,000-fold molar excess relative to a second-probe. In a preferred embodiment, the contacting step includes an analyte that is a nucleic acid, a protein, a lipid, a carbohydrate or a compound that is a combination thereof. Preferably, the contacting step includes an analyte that is a nucleic acid, and more preferably, the analyte is RNA. In another embodiment, at least one probe in the probe reagent is a nucleic acid, a protein, a lipid, a carbohydrate or a compound that is a combination thereof. Preferably, at least one probe is a nucleic acid or analog thereof. More preferably, at least one probe is a DNA, RNA, DNA analog, RNA analog or a molecule that includes a combination thereof.

Another aspect of the invention is a composition for use as a probe reagent for detecting an analyte in a single sample. This composition includes at least two probes, wherein each probe comprises a target binding moiety that binds specifically to a target region of the analyte, and a detectable label,
wherein each probe binds to a target region that differs from the target region of another probe in the probe reagent, and wherein each probe is present in an amount capable of detecting a range of analyte concentrations that differs from a range of analyte concentrations detectable by another probe in the reagent, such that the probe reagent is capable of detecting the analyte over an extended range of analyte concentrations that is greater than the range of analyte concentrations detectable by a single probe in the reagent and wherein a first probe in the probe reagent is present in an amount that differs by at least about a 10-fold molar excess relative to a second probe in the probe reagent. In another embodiment, each probe is distinguishable from another probe in the probe reagent because of its specific activity, a property resulting from its detectable label, or a combination thereof. Another embodiment of the composition includes, for each probe, a nucleic acid oligonucleotide having a target binding moiety that is a base sequence. In one embodiment, each probe has a detectable label that is an acridinium ester derivative. Preferably, each probe has a different detectable label, and each probe has a different specific activity compared to another probe in the reagent. In another embodiment, each probe is a nucleic acid oligonucleotide in which the target binding moiety binds specifically to a target region that is a nucleic acid base sequence, and preferably each probe has a detectable label that is an acridinium ester derivative.

### BRIEF DESCRIPTION OF THE DRAWINGS

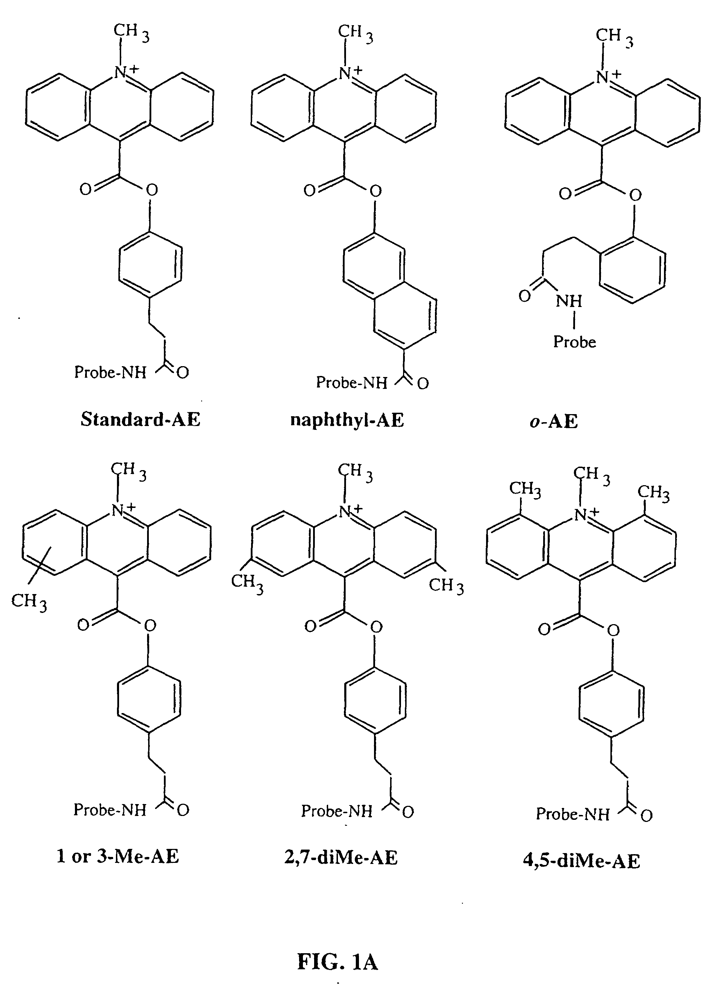

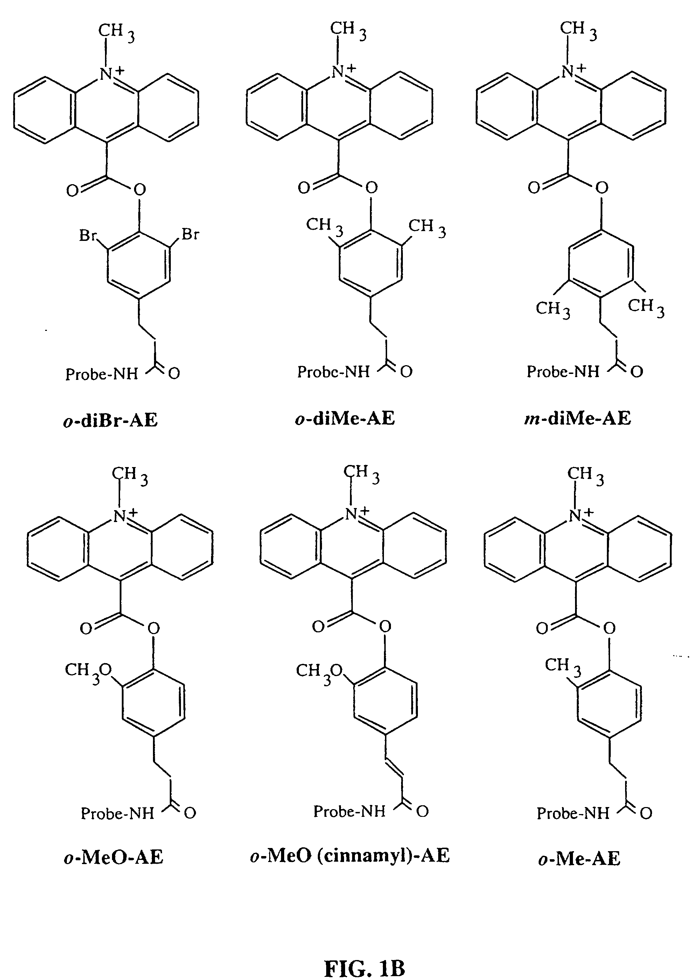

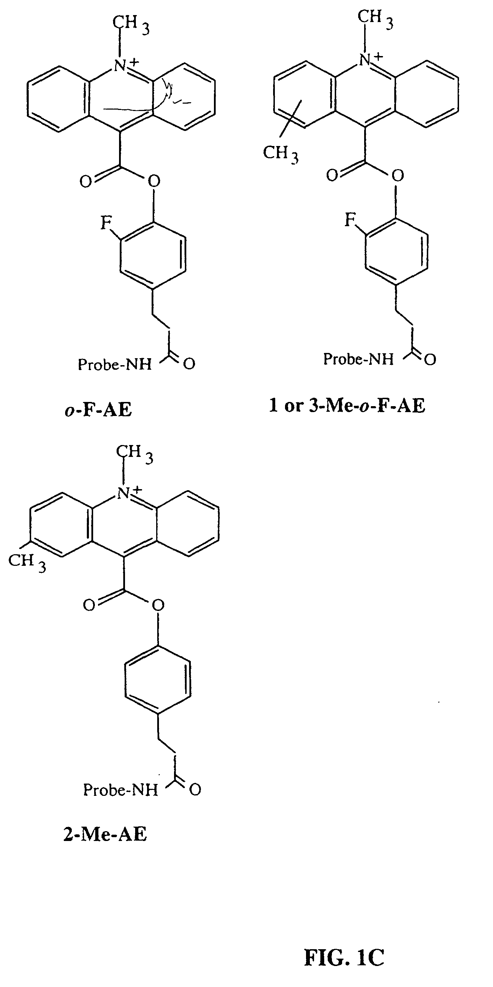

FIGS. 1A, 1B and 1C show the structures of a number of acridinium ester ("AE") derivatives, and their corresponding nomenclatures, which have different chemiluminescent reaction properties such that certain combinations of these labels may be used as distinguishable labels. The compounds shown are: in FIG. 1A, standard AE, naphthyl-AE, *ortho*-AE ("*o*-AE"), 1- or 3-methyl-AE ("1 or 3-Me-AE"), 2,7-dimethyl-AE ("2,7-diMe-AE"), and 4,5-dimethyl-AE ("4,5-diMe-AE"); in FIG. 1B, *ortho*-dibromo-AE ("*o*-diBr-AE"), *ortho*-dimethyl-AE ("*o*-diMe-AE"), *meta*-dimethyl-AE ("*m*-diMe-AE"), *ortho*-methoxy-AE ("*o*-MeO-AE"), *ortho*-methoxy(cinnamyl)-AE ("*o*-MeO(cinnamyl)-AE"), and *ortho*-methyl-AE ("*o*-Me-AE"); and in FIG. 1C, *ortho*-fluoro-AE ("*o*-F-AE"), 1- or 3-methyl-*ortho*-fluoro-AE ("1 or 3-Me-*o*-F-AE"), 1- or 3-methyl-*meta*-difluoro-AE ("1 or 3-Me-*m*-diF-AE"), and 2-methyl-AE ("2-Me-AE").

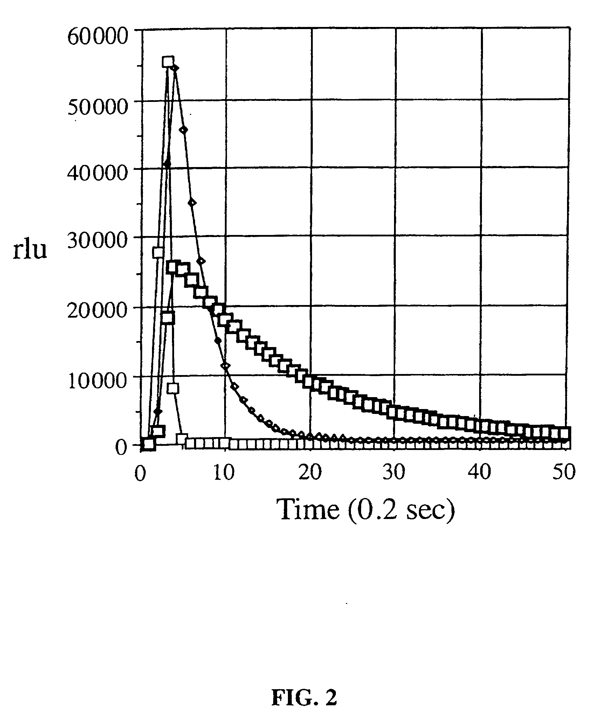

FIG. 2 is a plot of the kinetics of light emission for 1-Me-*m*-diF-AE (□), 1-Me-AE (◇) and *o*-MeO(cinnamyl)-AE (□) (where the y axis is relative light units ("RLU"), and the x axis is time intervals (in 0.2 sec), showing that 1-Me-*m*-diF-AE reacts most quickly, then 1-Me-AE reacts, and *o*-MeO(cinnamyl)-AE reacts over a longer period of time.

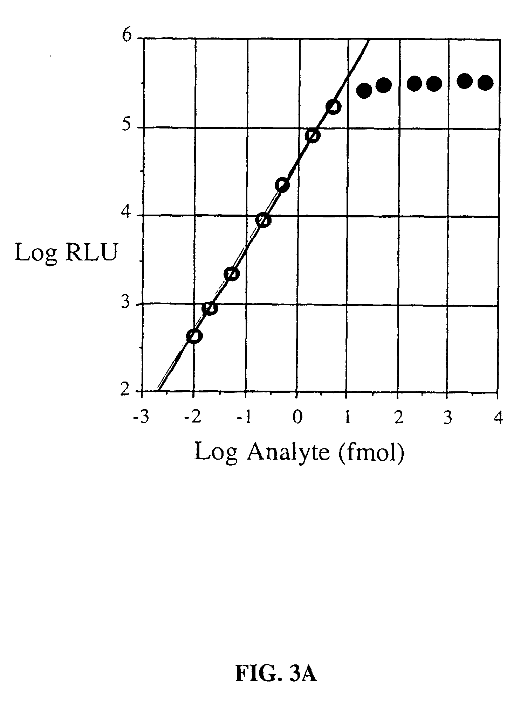

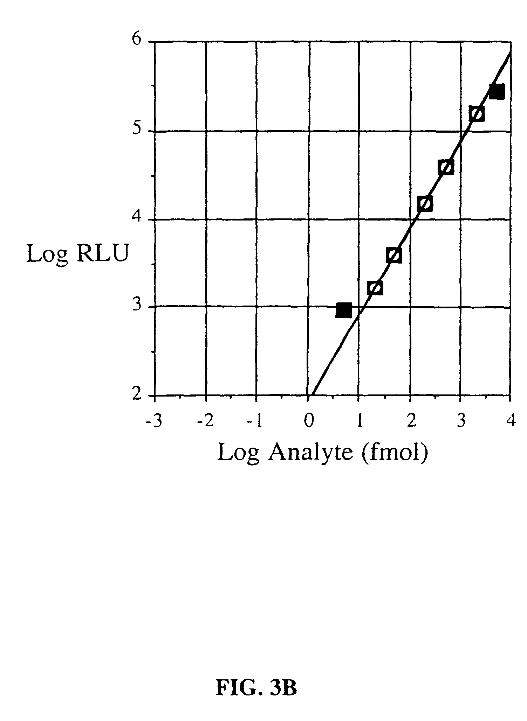

FIGS. 3A and 3B are log-log scale graphs showing the chemiluminescent signals (y axis, log RLU) obtained in separate experiments from two different and differently-labeled oligonucleotide probes (FIG. 3A for o-F-AE-labeled Probe 1 (**●**, ○), and FIG. 3B for 2-Me-AE-labeled Probe 2 (■,□ )), directed to the same target RNA analyte, as a function of target amount (x axis, log analyte in fmol); Probe 2 (FIG. 3B) was present at a specific activity of 100-fold less than that of the Probe 1 (FIG. 3A). Although all data points obtained are show, only some (○, 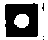 ) were used to calculate the best-fit line.

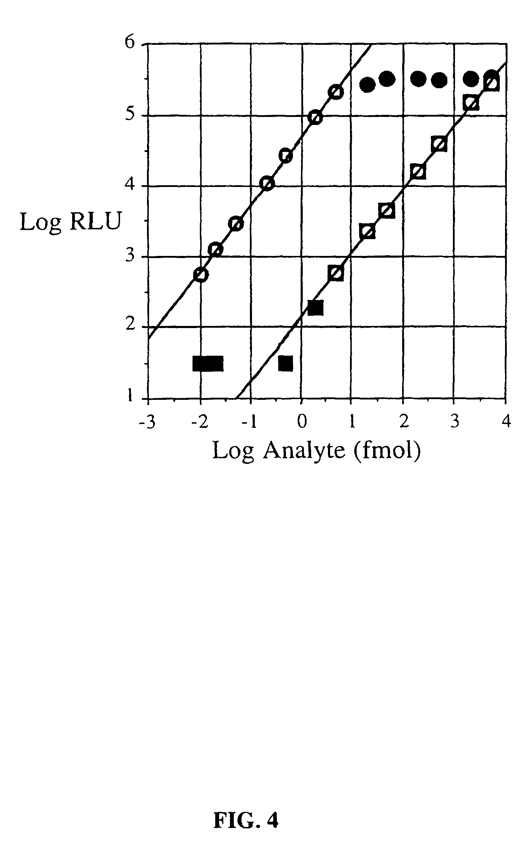

FIG. 4 is a log-log scale graph of chemiluminescent signals obtained using the same probes, labels and target as in FIGS. 3A and 3B, but the entire assay (probes in the same reaction mixture binding to analyte, and chemiluminescence) was performed in the same vessel; the symbols used for data points are as in FIGS. 3A and 3B.

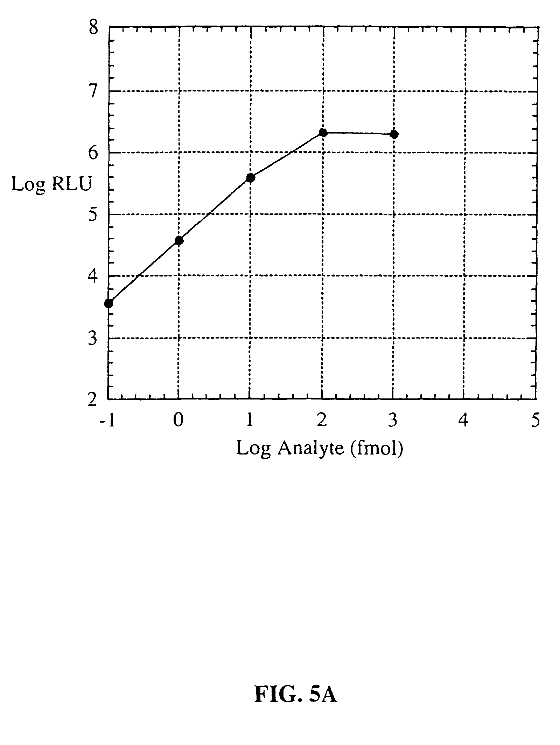

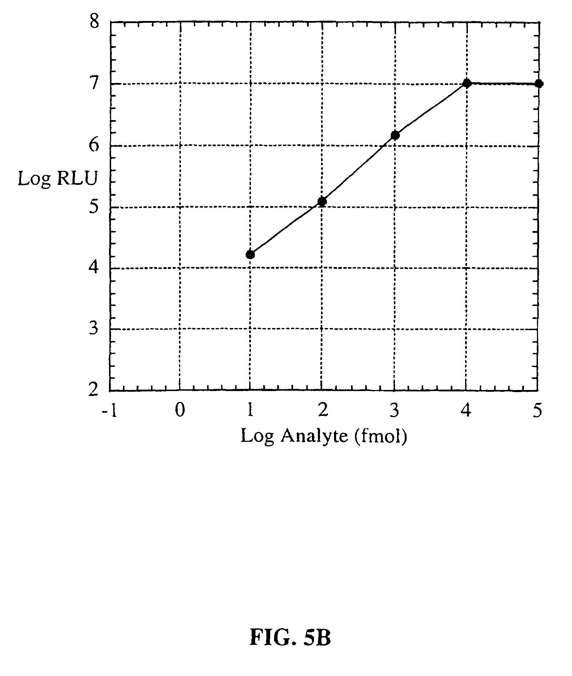

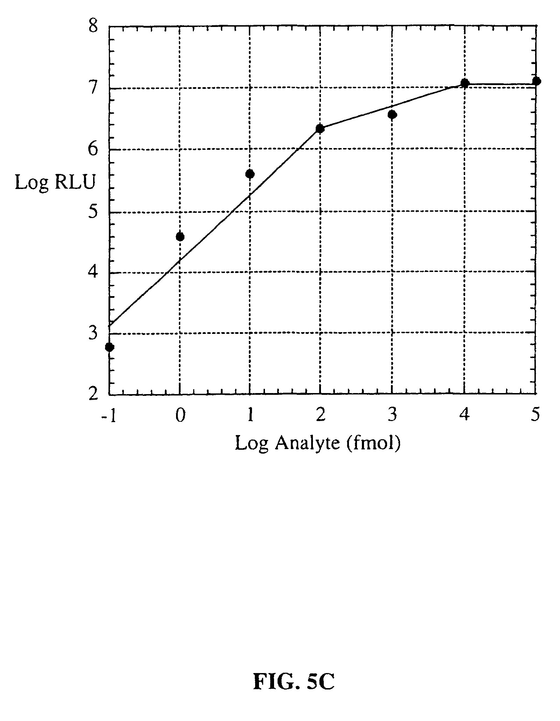

FIGS. 5A to 5C are log-log scale graphs showing the chemiluminescent signals (y axis, log RLU) as a function of target amount (x axis, log analyte in fmol) obtained in separate experiments using two different probes (Probes 3 and 4) labeled with the same chemiluminescent label and assayed using: Probe 3 alone (FIG. 5A), Probe 4 alone (FIG. 5B) and Probes 3 and 4 together (FIG. 5C).

### DETAILED DESCRIPTION OF THE INVENTION

The present invention is directed to methods and compositions permitting the detection of at least one analyte over a broader total concentration range than is otherwise commonly possible when using a single probe, or using multiple probes in other types of assays (e.g., as described by Taber in EP 114,668). Generally, the method utilizes two or more labeled probes, each probe targeted to the same analyte in a different target region. Each labeled probe is used to detect analyte within a different specified concentration range, and is present in the assay in an amount which corresponds in a defined manner to the maximum molar amount of analyte within that range. The probe reagent contains a first probe present in at least a 10-fold molar excess relative to a second probe. Thus, the labeled probes are present in the assay at different concentrations, which correspond to the different analyte concentration ranges sought to be detected. In one embodiment of the invention, the signals produced by each of the labels are detected by measuring the same type of property change (e.g., light detection). In a preferred embodiment, the signal produced by each label is independently distinguishable (e.g., by detecting different properties associated with each label, such as light and radioactive decay, or distinguishable measurements of the same property, such as light at different wavelengths). Furthermore, each probe may be labeled at the same, or preferably at different specific activities.

One objective of the invention is a method of detecting analytes and extending the range of concentrations at which said analytes can be detected, by using two or more probes. Each probe is labeled with a detectable label and directed to a different target region of the same analyte.

Preferably, the labels to which the probes are joined are separately detectable. By "separately detectable" is meant that the labels can be present in the same reaction vessel and each distinguished in the presence of the other. Such labels may be of the same general type, such as radionuclides (for example, ³²P and ¹²⁵I; the first being detectable by emission of β particles and the other by emission of γ rays), chemiluminescent labels and fluorescent labels. Alternatively, one probe may be labeled with a particular type of label and another probe may be labeled with a different type of label, such as the first probe with radionuclide and the second probe with a fluorescent label. In other embodiments, the labels on the probes are not separately distinguishable (i.e., the same measurable property is detected), so long as they are present on each probe at different specific activities.

By "labeled" is meant that a probe is joined to a labeling compound. The label can be joined either directly or indirectly. An example of indirect labeling is through the use of a bridging molecule, such as a secondary antibody or a bridging oligonucleotide, which is itself either directly or indirectly labeled. Direct labeling can occur through covalent bonds or through non-covalent interactions such as hydrogen bonding, hydrophobic and ionic interactions, or through the formation of chelates or coordination complexes.

When reference is made to the concentration or amount of a particular probe corresponding to an amount of analyte which the probe is designed to detect, it will be understood that by "corresponding to" is meant that the concentrations or amounts of the probe and analyte are related in a predictable and reproducable way; for example, by a particular ratio, under a set of assay conditions.

By "oligonucleotide" or "oligomer" is meant a multimeric compound comprised of nucleosides or nucleoside analogs which have nitrogenous bases, or base analogs, able to specifically bind a nucleic acid analyte to form a stable probe:analyte complex. The nucleosides may be linked together by phosphodiester bonds to form a polynucleotide, or may be linked in any other manner that permits the formation of a target-specific complex with a target nucleic acid. Other linkages may include, for example, phosphorothioate linkages, methylphosphonate linkages, and peptide bonds. Peptide nucleic acids, as used herein, are included in the compounds referred to as oligonucleotides. Sugar moieties (ribose, deoxyribose) may be substituted with groups affecting stability of the hybridization reaction (but not preventing formation of a probe:analyte complex), as, for example, with 2' methoxy substitutions and 2' halide substitutions, such as 2'-F. The bases may be conventional bases (A, G, C, T or U) or analogs thereof (see e.g., *The Biochemistry of the Nucleic Acids* 5-36, Adams et al., ed., 11^{th} ed., 1992).

By "specific activity" is meant units of detectable signal obtained from a label per unit measurement of probe. The units of detectable signal may be expressed in counts per minute (cpm), light absorbance units at a specified wavelength, relative light units ("RLU"), units of enzymatic activity or any other unit of measuring the amount of label present. Likewise, the units of probe measurement may be expressed as units of mass (e.g., µg), or as a measurement of the number of molecules (e.g., µmoles).

By the terms "binding partner" or "probe" is meant a molecular compound capable of binding to or interacting with an analyte. A binding partner or probe is used to indicate the presence or amount of analyte in a sample. Unless the binding partner or probe is inherently or directly detectable, a label is directly or indirectly conjugated to the probe. Analytes which may be detected include, for example, nucleic acids, antibodies, proteins, enzymes, lipids, carbohydrates, cell-surface receptors, cytokines, hormones, antigens, metals, molecular complexes such as polymeric arrangements of proteins or other macromolecules, and the like. Likewise, binding partners or probes for detecting such analytes may include, for example, nucleic acid probes, antibodies, antigens, haptens, chelating agents, enzymes, enzyme substrates, proteins and analogs of these.

By "target region" or "region" is meant any portion of an analyte, continuous or discontinuous, that binds specifically to a probe or binding partner. For example, when the analyte is a nucleic acid, a target region may comprise a nucleotide base sequence that binds specifically a probe. The target region may also comprise a secondary or tertiary structure of the analyte to which a probe specifically binds. In another example, when the analyte is a protein or peptide. a target region may be an amino acid sequence or a conformational domain of the protein or peptide to which the probe specifically binds. Preferably, a target region does not overlap another target region of the same analyte, so that simultaneously contacting multiple probes to the analyte will not result in competition between probes.

It is an object of the invention to provide methods for the detection of a wide range of target analyte concentrations. Typically, the methods of the present invention increase the ability to accurately detect the analyte by at least two orders of magnitude. By using additional probes with separately distinguishable labels, the dynamic range of an assay of a single sample may be extended by 3 to 6 orders of magnitude or more. These methods allow analyte detection in a single sample without performing sample dilutions or testing replicate samples under different conditions. Preferably, the dynamic response of the assay to an expected analyte level is designed *a priori* to cover a range of analyte amounts. The method preferably allows for sample analysis in a single tube.

A further object of the invention is to provide compositions for the detection or quantification of an analyte that can be used by a person with a minimum of specialized training in medical, clinical, or scientific methodology. By permitting the testing of an analyte which may be present in a sample at a concentration within a wide range of potential concentrations, the invention provides compositions which can permit the automation of many aspects of assays while minimizing sample handling and risk of error. Thus, the present invention provides compositions that minimize the level of laboratory personnel training needed to conduct assays, permit assay automation and reduce variability of results.

A further object of the present invention is to provide a method for detecting or measuring one or more analytes present in a sample that includes the steps of contacting the sample with a probe reagent that includes two or more probes, under conditions favoring the binding of the probes with the analyte. Each probe is joined to a label and specifically binds to a separate target region of the analyte, such that each labeled probe is designed to detect the analyte over a different range of analyte concentrations and the amount of each probe present in the probe reagent corresponds to the amount of the analyte sought to be detected by that probe. Then, the method includes the step of detecting the presence of at least one label as an indication of the presence or amount of the analyte in the sample, wherein the range of possible analyte amounts in the sample capable of being detected or measured by the probe reagent is greater than the range of analyte amounts in the sample capable of being detected or measured by one labeled probe or a combination of probes as described in the prior art (e.g., see EP 114,668). Preferably, both the contacting and detecting steps are performed in the same vessel. In one embodiment, the method makes use of separately distinguishable labels. In another embodiment, the method makes use of two or more labeled probes having different specific activities.

Probes are present in the probe reagent in different amounts. The probe reagent contains a first probe present in at least a 10-fold molar excess relative to a second probe. The amount of each probe corresponds to and defines the upper limit of the range of concentrations of the analyte that the labeled probe specifically binds to and detects. This feature is important because the amount of each labeled probe in the assay can define the amount of background present. Also, when the probes differ in their specific activities, the probe having the highest specific activity is generally present in the lowest amount, while the probe having the lowest specific activity is generally present in the highest amount. Thus, background from label associated with unbound probe is minimized.

Another object of the invention provide compositions and kits for detecting analytes which may be present in a sample within a wide range of possible concentrations. Such compositions include a probe reagent that includes two or more labeled probes capable of binding to separate target regions of the analyte. The labeled probes in the reagent can detect the analyte over a different range of possible analyte concentrations because the concentration of each probe in the reagent corresponds to the maximal concentration of the analyte to be detected by that particular probe. An analyte is capable of binding two or more probes, and each probe is capable of specifically binding to a target region of the analyte. A probe and/or analyte may be a nucleic acid or oligonucleotide, protein (e.g., an antigen, antibody, hormone, cytokine or cellular receptor), or a peptide nucleic acid.

The present invention includes methods, kits, and compositions for the detection and measurement of an analyte which may be present in a sample within a wide concentration range. The method obviates the need to dilute samples or make replicate tests of a single sample under different conditions. The invention provides a cost-, time-, and labor-effective format for the detection of an analyte capable of binding two or more binding partners.

The invention involves three observations common to analyte detection assays. The first observation is that any assay system employing a label for analyte detection yields a background level of non-specific signal contributed by "free" label. By "free" label is meant label that is present in the assay in an unbound form after the assay has been performed, or labeled probes that have failed to bind analyte and yield detectable signal. Assay formats typically have an inherent background level which may vary from assay to assay, but is typically in the range of about 0.01% to about 10%, or more preferably in the range of about 0.01% to about 1%, of the total signal measured in the assay. Background may also include electronic noise present in the instrument used to detect the label.

The second observation is that each instrument or method used to detect label has a maximum level of signal detection, beyond which additional signal is not detected. For example, a luminometer may have a maximum detection level of 2 million relative light units ("RLU") and if a sample contains label amounting to 4 million RLU is read in this instrument, it will underestimate the amount of label present in the sample by half (i.e., it will indicate 2 million RLU). This phenomenon is described as loss of dynamic range at the "top end." When combined with the first observation, it can be readily seen that there are limits on both the minimum and the maximum levels of analyte detectable in a standard assay system.

The third observation is that different labels can be detected separately in the presence of each other. Thus, the background contributed by a first label will not usually substantially interfere with detection of another independently-detectable label. Although no interference between different labels is advantageous, insubstantial interference does not prevent independent detection of two or more labels.

Background contributed by labeled probes poses a problem when different probes cannot be distinguished. Background contributed by one probe present in high amounts may obscure low amounts of signal from another probe present in low amounts. This problem can be resolved in two ways, as described in detail below. First, probes having different specific activities can be used, such that one labeled probe present in high amounts will not create significant background if its specific activity is low. Second, if labeled probes having the same specific activities are independently discernable, the background contributed by one labeled probe will not overwhelm the signal obtained by another probe present in low amounts.

When populations of different probes are each targeted to separate target regions of a single analyte, the different probes will not compete with each other for analyte binding. Thus, the different probes separately bind to and indicate the presence of the analyte. When labeled probes are used, the separate detection of each target region is possible because each probe:target region complex is an independent indication of the presence of analyte. By supplying each labeled probe in a mixture at a different concentration from each other labeled probe, each target region is an indication of an analyte concentration within a range of concentrations. Thus, a collection of such probes constitutes a sequence of concentration ranges which can be detected.

The methods of the present invention employ similar but different modes of expanding the concentration range of an assay that detects an analyte using more than one probe or binding partner. To more fully illustrate this, three model systems are described below.

### Model System 1: Use of Indistinguishable Labels on Probes Having Different Specific Activities

For the purposes of this illustration, three probes are used, each targeted to different target regions of the same analyte. It will be understood that any number of probes greater than one would be equally useful in other applications of the method. Each probe is labeled with a single type of label. In practice, this label could be different from probe to probe, such as different radionuclides or different fluorescent molecules, but the signal given off by each label is presumed to be indistinguishable from the signal given off by the other labels.

In this model assay, the mixture of labeled Probes 1, 2 and 3 contact the analyte under conditions that favor binding of all the probes to their respective target regions on the analyte. After binding, a step of distinguishing the bound probes from unbound probes is included; such a step can be readily determined by one skilled in the art depending on whether a heterogeneous or homogeneous assay system is used (e.g., a physical separation step, or a hydrolysis step that selectively inactivates label on unbound probe). Then, the bound labeled probes are each detected under appropriate conditions, which can be readily determined by one skilled in the art depending on the labels used (e.g., by detecting light, radioactive decay or products of an enzyme-substrate reaction). The calculations that follow assume 100% efficiency of detection.

In all of the Model Systems presented, it will be assumed that each probe is targeted to a target region which is present in a single copy for each unit of analyte, e.g., a nucleotide sequence region unique to a target nucleic acid, or a domain unique to a target polypeptide. One skilled in the art will appreciate that a plurality of such sites may exist for each target region for which a particular probe is designed to bind. Although the calculations which follow assume a one-to-one correspondence between target regions and analyte, in the case where two or more target regions are present for each unit of analyte, these calculations would differ only in requiring a simple correction to account for this fact. That is, one skilled in the art will realize that the calculations provided below should be multiplied by two for analytes that contain two target regions per analyte, by three for analytes that contain three target regions per analyte, and so forth.

For this calculation, assume that the desired detectable analyte amounts range from 0.01 fmole (1 fmole equals 10⁻¹⁵ moles) to 10,000 fmoles. Three probes at different concentrations are used in the assay: 1 fmole of Probe 1, 100 fmoles of Probe 2, and 10,000 fmoles of Probe 3. These probes are labeled at different specific activities that are inversely related to the amount of probe in the assay. For this illustration, assume that Probe 1 is labeled at 10⁸ units of label/pmole (10⁻¹² moles) of probe, Probe 2 with 10⁶ units of label/pmole and Probe 3 with 10⁴ units of label/pmole. This results in the same amount of label (10⁵ units) being contributed to the reaction by each probe.

For this illustration, assume that when no analyte is present in the sample, the background is 0.1% of the total detectable signal in the assay. Based on the above, this amount is 0.1% x 10⁵ units = 100 units of background for each probe, totaling 300 units for all three probes. Those skilled in the art will appreciate that this relatively low amount of background permits detection of analyte at low concentrations.

For example, if 3 attomoles (1 attomole = 10⁻¹⁸ moles) of analyte are present in the assay, 3 attomoles of Probe 1 bind, and the Probe 1:analyte complex produces a signal of 300 units. When combined with the assay background, the overall signal detected is 600 units (300 units of background and 300 from Probe 1:analyte complex), yielding a two-fold signal-to-noise ("S/N") ratio at this analyte concentration; a two-fold S/N ratio or greater is generally accepted by those skilled in the art as a positive signal. When 3 attomoles of analyte are present, 3 attomoles each of Probe 2 and Probe 3 also bind the analyte. However, due to their lower specific activities, Probe 2 contributes only 0.3 units of signal, while Probe 3 contributes 0.0003 units. Therefore, the signals from these probes do not contribute significantly to the signal obtained from Probe 1 at this analyte concentration.

Using the same probes, if the concentration of analyte is increased to 1 fmole, all of Probe 1 binds to the analyte, and the Probe 1:analyte complex results in 10⁵ units of detected label. Only 0.1% of 10⁵ units of signal from Probe 1 (100 units) is due to background for this probe. Also in this assay, 1 fmole of Probes 2 and 3 bind to the analyte to produce Probe 2:analyte and Probe 3:analyte complexes. Because these probes are labeled at a lower specific activity, the Probe 2 label produces 1,000 units of signal and Probe 3 label produces 10 units of signal upon detection. That is, Probe 2 label contributes approximately 1% of the total signal detected and Probe 3 label is below background signal. The total signal detected when 1 fmole of analyte is present is 100,000 + 1000 + 10 = 101,010 units.

When between 1 and 100 fmole of analyte are present in the assayed sample, the detectable signal from labeled Probe 2 varies in proportion to target concentration. At such concentrations, all of Probe 1 is bound to its target region, producing a signal equivalent to its 100,000 units of detectable label. The label in Probe 2:analyte complexes is also detected. For example, at 100 fmoles of analyte, all of the available Probe 2 is bound, and the Probe 2 label is detected as another 100,000 units of signal; 100 fmoles of Probe 3 is also bound, contributing 1,000 units of label. Thus, 100 fmoles of analyte is detected as 100,000 + 100,000 + 1,000 = 201,000 units of signal.

Similarly, in a range of 100 fmoles to 10,000 fmoles of analyte, all of Probe 1 and Probe 2 in the reaction mixture are bound to analyte producing 100,000 units of detectable signal for each. Probe 3 is available to bind with up to 10,000 fmoles of analyte, producing signal from Probe 3:analyte complexes proportionate to the amount of labeled Probe 3 that is bound. For example, when 10,000 fmoles (or 10 pmoles) of analyte are present, Probes 1, 2, and 3 each contribute 100,000 units of detectable signal, totaling 300,000 units of detectable signal.

The calculated contributions of units of signal for each of labeled Probes 1, 2 and 3, and total signal, for different amounts of analyte in an assay as discussed above are presented in Table 1. From the calculated amounts presented in Table 1, it can be seen that the use of three different probes extends the assay's range beyond that available using only one probe because the detected signal is cumulative of the contributions of the three independent probes. This would not be true if the three probes were identical or otherwise targeted to the same target region of the analyte; in this case, each probe would compete for binding with each other probe, and the mixture of such probes would essentially constitute an "averaging" of the specific activities of the three probes, with the higher abundance, low specific activity probe predominating.

**TABLE 1**

| **Amount of Target to be Detected (pmoles)** | **Probe 1 Units of Signal** | **Probe 2 Units of Signal** | **Probe 3 Units of Signal** | **Total Units of Signal** |
|---|---|---|---|---|
| 0.00001 | 1,000 | 10 | 0.1 | 1,010.1 |
| 0.0001 | 10,000 | 100 | 1 | 1,011 |
| 0.001 | 100,000 | 1,000 | 10 | 101,010 |
| 0.01 | 100,000 | 10,000 | 100 | 110,100 |
| 0.1 | 100,000 | 100,000 | 1,000 | 201,000 |
| 1 | 100,000 | 100,000 | 10,000 | 210,000 |
| 10 | 100,000 | 100,000 | 100,000 | 300,000 |

### Model System 2: Use of Distinguishable Labels on Probes Having the Same Specific Activity

As in Model System 1, three probes are used and each probe is targeted to a distinct region of the analyte, such that the probes do not compete for binding to the analyte. In this Model System, each probe is labeled with a different, separately distinguishable label and the probes are labeled at the same specific activity. It will be appreciated by those skilled in the art that these labels may be of the same or of different types. For example, all the labels may be chemiluminescent labels, or alternatively, one label may be a radioactive label, one a fluorescent label, and one a chemiluminescent label. Appropriate detection systems that are specific for each of the labels are well known to those skilled in the art and require only routine testing to optimize detection.

The same basic assay system and range of analyte concentrations to be detected are used as in Model System 1. Also, the probes are present in different amounts to correlate with the different analyte concentration ranges detectable for each probe. Thus, for this illustration, 1 fmole of Probe 1 is present, 100 fmoles of Probe 2 is present, and 10,000 fmoles of Probe 3 is present. Because each probe is labeled at the same specific activity (here arbitrarily chosen to be 10⁸ units of label per pmole of probe), there are 10⁵ units of detectable signal from labeled Probe 1, 10⁷ units of detectable signal from labeled Probe 2, and 10⁹ units of detectable signal from labeled Probe 3 in the reaction mix.

For this illustration, the background is again arbitrarily set at 0.1% of the maximum detectable signal inherent in the assay. Unlike those described in Model System 1, the labels in this Model System are distinguishable. Thus, when 1 fmole of analyte is present in the sample, all of Probe 1 binds to the analyte and resulting in 10⁵ units of signal from Probe 1:analyte complexes; and the background for Probe 1 will be 100 units of signal. At this same analyte concentration, 1 fmole of Probes 2 and 3 also bind to the target, producing 10⁵ units of signal from each of their labels. Because the label for each probe is independently detectable, neither Probe 2 or 3 is detected when Probe 1 is specifically detected, and vice versa. Similarly, the background contributed by labels 2 and 3 generally will not affect the detection of label 1, and vice versa, because of the specific and independent detection methods used.

At amounts greater than 1 fmole of analyte, no additional Probe 1 is available to bind because only 1 fmole of Probe 1 is present in the reaction. For example, when 100 fmoles (0.1 pmole) of analyte is present, all of Probe 1 is bound to analyte and 100 fmoles of each of Probes 2 and 3 are bound to their respective target regions of the analyte, producing 10⁵ units of signal from Probe 1:analyte, and 10⁷ units of signal from each of Probe 2:analyte and Probe 3:analyte complexes. Background from each of the labels associated with Probes 2 and 3 is 0.001 x 10⁷ units, or 10,000 units. Again, because the labels are independently detectable, the background contributed by one label does not usually interfere with detection of the others.

At analyte concentrations of between 100 and 10,000 fmoles, Probes 1 and 2 are bound completely to analyte, producing 10⁵ units of signal from Probe 1;analyte and 10⁷ units of signal from Probe 2:analyte complexes detected. Probe 3 binds up to 10⁵ fmoles (10 pmoles) of analyte; because this probe is labeled at the same specific activity as the other probes, 10⁹ units of signal from Probe 3 are detected. The background contributed by the label of Probe 3 is 10⁹ x 0.001, or 10⁶ units of signal.

The calculated units of signal for each of labeled Probes 1, 2 and 3, and total signal for each detectable label, for different amounts of analyte in an assay as discussed above are presented in Table 2.

**TABLE 2**

| **Amount of Target to be Detected (pmoles)** | **Probe 1 Units of Signal** | **Probe 2 Units of Signal** | **Probe 3 Units of Signal** | **Total Units of signal** |
|---|---|---|---|---|
| **0.00001** | 10³ | 10³ | 10³ | 10³ units from Label 1 |
| | | | | 10³ units from Label 2 |
| | | | | 10³ units from Label 3 |
| **0.0001** | 10⁴ | 10⁴ | 10⁴ | 10⁴ units from Label 1 |
| | | | | 10⁴ units from Label 2 |
| | | | | 10⁴ units from Label 3 |
| **0.001** | 10⁵ | 10⁵ | 10⁵ | 10⁵ units from Label 1 |
| | | | | 10⁵ units from Label 2 |
| | | | | 10⁵ units from Label 3 |
| **0.01** | 10⁵ | 10⁶ | 10⁶ | 10⁵ units from Label 1 |
| | | | | 10⁶ units from Label 2 |
| | | | | 10⁶ units from Label 3 |
| **0.1** | 10⁵ | 10⁷ | 10⁷ | 10⁵ units from Label 1 |
| | | | | 10⁷ units from Label 2 |
| | | | | 10⁷ units from Label 3 |
| **1** | 10⁵ | 10⁷ | 10⁸ | 10⁵ units from Label 1 |
| | | | | 10⁷ units from Label 2 |
| | | | | 10⁸ units from Label 3 |
| **10** | 10⁵ | 10⁷ | 10⁹ | 10⁵ units from Label 1 |
| | | | | 10⁷ units from Label 2 |
| | | | | 10⁹ units from Label 3 |

As can be seen from Table 2, unlike the situation illustrated in Model System 1 in which the labels are indistinguishable and of different specific activities, in this embodiment the same linear relationship is maintained between the amount of analyte in the sample and the amount of signal detected, for each of the three probes used. Although the assay methodology of Model System 2 is valid, it is possible that a detection device or method may not measure signals within all of the ranges presented in Table 2. For example, if the detection devices or methods for each label do not accurately measure the upper limits of signal for each label (i.e., 100,000 units for Label 1, 10,000,000 units for Label 2, and/or 1,000,000,000 units for Label 3), then the accuracy of this approach is lessened. It will be appreciated that the method or device used to detect one label need not be the same device or method used to detect another said label.

If the detection methods and/or devices are capable of accurately measuring signal generated from each label throughout its "effective detection range" (i.e., the range of analyte amounts which depend on the measurement of that particular label in the assay), and each label is substantially distinguishable from each other label, then this assay format has distinct advantages over the format illustrated in Model System 1. Because the specific activities of the labels in Model System 2 are the same, the same relationship exists between the amount of analyte and the amount of signal generated throughout the dynamic range of the assay for all the probes. Thus, this assay format permits detection of analyte with enhanced sensitivity compared to that of Model System 1.

### Model System 3: Use of Distinguishable Labels on Probes Having Different Specific Activities

This Model System describes detecting analyte across a broad range of potential analyte amounts or concentrations by using probes labeled with distinguishable labels that are joined to different probes, where each probe at a different specific activity. Thus, this Model System is a hybrid of the conditions of Model Systems 1 and 2 which previously described the features in more detail.

As in Model Systems 1 and 2, to illustrate Model System 3, three labeled probes are used, each specific for a different target region of the analyte. As before, each probe is present in a different amount (1 fmole of Probe 1, 100 fmoles of Probe 2, and 10,000 fmoles of Probe 3), allowing each probe to detect a specific range of analyte in a sample. As in Model System 1, each probe is labeled at a different specific activity compared to the other probes: Probe 1 has a specific activity of 10⁸ units of signal per pmole, Probe 2 has a specific activity of 10⁶ units of signal per pmole, and Probe 3 has a specific activity of 10⁴ units of signal per pmole. As a result, the maximum amount of each probe in the assay equals 10⁵ units of signal. As in Model System 2, each probe is separately distinguishable.

The amounts of detectable signal for each probe in assays containing different amounts of analyte were calculated as discussed in Model Systems 1 and 2 above. Table 3 shows the calculated units of signal for each of labeled Probes 1, 2 and 3, and total signal for each detectable label, for different amounts of analyte (in pmols) in an assay as discussed for Model Systems 1 and 2 above.

**TABLE 3**

| **Amount of Target to be Detected (pmoles)** | **Probe 1 Units of Signal** | **Probe 2 Units of Signal** | **Probe 3 Units of Signal** | **Total Units of Signal** |
|---|---|---|---|---|
| **0.00001** | 10³ | 10 | 0.1 | 10³ units from Label 1 |
| | | | | 10 units from Label 2 |
| | | | | 0.1 units from Label 3 |
| **0.0001** | 10⁴ | 10² | 1 | 10⁴ units from Label 1 |
| | | | | 10² units from Label 2 |
| | | | | 1 units from Label 3 |
| **0.001** | 10⁵ | 10⁴ | 10 | 10⁵ units from Label 1 |
| | | | | 10⁴ units from Label 2 |
| | | | | 10 units from Label 3 |
| **0.01** | 10⁵ | 10⁴ | 10² | 10⁵ units from Label 1 |
| | | | | 10⁴ units from Label 2 |
| | | | | 10² units from Label 3 |
| **0.1** | 10⁵ | 10⁵ | 10³ | 10⁵ units from Label 1 |
| | | | | 10⁵ units from Label 2 |
| | | | | 10³ units from Label 3 |
| **1** | 10⁵ | 10⁵ | 10⁴ | 10⁵ units from Label 1 |
| | | | | 10⁵ units from Label 2 |
| | | | | 10⁴ units from Label 3 |
| **10** | 10⁵ | 10⁵ | 10⁵ | 10⁵ Units from Label 1 |
| | | | | 10⁵ units from Label 2 |
| | | | | 10⁵ units from Label 3 |

Because each label is separately distinguishable from the other labels, each probe is detected without being overwhelmed by signal from the other probes. Because the specific activities of each of the labeled probes are different, the amount of signal obtained while detecting very small amounts of analyte is enhanced, while the amount of signal obtained while detecting very large amounts of analyte is attenuated. This feature is particularly advantageous when the detection method or device used to detect one or more of these labels is limited to a particular dynamic range. For example, in the presently illustrated situation, each of the three probes yields detectable signal in the same range of about 10³ to 10⁵ units of signal. Thus, while the assay detects analyte over six orders of magnitude, a detection device used to detect any one probe:analyte complex only requires accuracy over two orders of magnitude.

This latter fact has advantages in particular aspects of the present invention. For example, separately distinguishable labels may be detectable using a single instrument or detection method. In particularly preferred aspects of the invention disclosed below, separately detectable chemiluminescent labels are used. Specific chemiluminescent labels may be detected by measuring the emission of light in the same wavelength range over different periods of time, or over the same period of time using mathematical methods to resolve the signals, using a single luminometer. Additionally, the labels are present at different specific activities, which decreases the luminometer dynamic range necessary to detect analytes over a wide range of concentrations. Therefore, any inherent limitations in the dynamic range of the luminometer do not limit the ability of the method to detect analyte over a greater concentration range than would be possible with a single probe, because separately detectable labels are used to cover this enhanced range, with each label being individually detectable within the intrinsic dynamic range of the instrument.

The following examples illustrate some of the preferred embodiments of the invention.

### EXAMPLE 1

### Extended Dynamic Range of Detection in Nucleic Acid Hybridization Assays

Although the methods of the invention are not limited to any particular format or use of a particular label type, this example is related to nucleic acid hybridization assays that use oligonucleotide probes labeled with chemiluminescent acridinium ester labels.

The ability of various luminescent, fluorescent, and chemiluminescent labels to be separately detected from each other in a mixture has been described in detail previously, e.g.,Woodhead et al., US Pat. No. 5,656,207 and Nelson et al., US Pat. No. 5,658,737. The latter reference, in particular, describes the use of different acridinium ester (AE) derivatives in a single assay and their ability to be separately detected in a number of formats. These formats include using chemiluminescent AE derivatives which emit light at different wavelengths, and separately detecting each label; using AE derivatives which react in a light emitting reaction at different rates and separately detecting the labels on the basis of reaction kinetics; and employing labels which react under different reaction conditions, such as pH, temperature, and the like.

FIGS. 1A to 1 C show the structures of several acridinium aryl ester compounds to which a linker moiety is attached; these acridinium ester ("AE") compounds are indicated by a shorthand nomenclature as: standard-AE, naphthyl-AE, *o*-AE, 1 or 3-Me-AE, 2,7-diMe-AE, 4,5-diMe-AE, *o*-diBr-AE, *o*-diMe-AE, *m*-diMe-AE, *o*-MeO-AE, *o*-MeO(cinnamyl)-AE, *o*-Me-AE, *o*-F-AE, 1 or 3-Me-*o*-F-AE, and 1 or-3-Me-*m*-diF-AE (the complete names of the compounds are listed in the Brief Description of FIGS. 1A to 1C). It will be understood that when these terms are used herein, they refer to the corresponding acridinium aryl esters represented in FIGS. 1A to 1C. The compounds 1-Me-AE, 1-Me-*o*-F-AE, and 1-Me-*m*-diF-AE are present in a mixture with their 3-methyl isomers; as used in this application, these nomenclatures will be understood to mean a mixture of the corresponding 1- and 3-methyl derivatives. *o*-MeO(cinnamyl)-AE is also sometimes referred to as "*o*-MeO(c)-AE". These compounds, their characteristics and preparation methods are described in detail in European Pat. App. No. 0709466. One skilled in the art will appreciate that these terms refer to the chemiluminescent acridinium ester compounds themselves, and that the illustrated linkers to which they are attached are generally interchangeable with other linkers known in the art, such as, for example, linkers having a longer or shorter chain length.

To illustrate how distinguishable labels may be detected in the present invention, three of the above-mentioned labels were used: 1-Me-*m*-diF-AE, 1-Me-AE, and *o*-OMe(c)-AE. The signals emitted by these compounds are capable of being independently distinguished on the basis of their reaction kinetics. As shown in FIG. 2,1-Me-*m*-diF-AE emits light extremely quickly after initiation of a chemiluminescent reaction; 1-Me-AE, has somewhat slower reaction kinetics, reaching a peak of light emission more slowly and emitting light over a longer time period than that of 1-Me-*m*-diF-AE; and *o*-OMe(c)-AE has the slowest reaction kinetics of the three, emitting light over a longer period of time than either of the other compounds.

FIG. 2 shows a plot of the light emission of each of these compounds, measured in relative light units (RLU), versus time (in 0.2 sec intervals). The chemiluminescent reaction was initiated by simultaneously reacting all the labeling compounds with a triggering agent (alkaline hydrogen peroxide). FIG. 2 shows that the majority of light emitted from 1-Me-*m*-diF-AE is produced in the first half second of reaction. 1-Me-AE emits light between 0 and 3 sec, after which very little additional light is produced; during the period between 0.5 and 3 sec, very little interference is contributed by the 1-Me-*m*-diF-AE emission. *o*-OMe(cinnamyl)-AE continues to emit light for at least 5 sec after the 3-sec time point.

These labels are distinguished experimentally by measuring the light emitted during three defined time "windows", one window for each label. One preferred set of windows for these compounds is: 0.0 to 0.6 sec for detection of 1-Me-*m*-diF-AE, 1.0 to 3.6 sec for detection of 1-Me-AE and 4.0 to 10 sec for detection of *o*-OMe(cinnamyl)-AE. Also, because the time course of reaction for each compound is constant, it is possible to predict and subtract light emitted from the other labels in a window used for the measurement of a particular label's signal through a reiterative statistical calculation. Such a statistical calculation, as performed for two labels, is provided in Nelson et al., US Pat. No. 5,658,737. One skilled in the art is capable of using such a calculation for the analysis and correction of signal obtained from three or more labels, as illustrated by the calculated Units of Signal contributed by individual labeled probes in Model System 3 above.

### EXAMPLE 2

### Comparison of Predicted Signal and Experimentally Determined Signal Using Distinguishable Labels

In this example. three AE derivatives (1-Me-*m*-diF-AE, 1-Me-AE, and *o*-OMe(cinnamyl)-AE) suitable for use in a homogeneous assay method (e.g., as described in US Pat. No. 5,283,174) are shown to be distinguishable under conditions that replicate those of analyte detection using chemiluminescence.

The 1-Me-*m*-diF-AE label (Label 1) was used at a relatively high specific activity of 10⁸ RLU/pmole, which is useful in the detection of analyte at the low end of the concentration range; the 1-Me-AE label (Label 2) was used at a specific activity of 10⁶ RLU/pmole; and the *o*-OMe(cinnamyl)-AE label (Label 3) was present at a specific activity of 10⁴ RLU/pmole. Other details of the labels and the assay protocol are shown below in Table 4. The actual amount of RLU added for the second and third labels was somewhat greater than 10⁵ to assure that 10⁵ RLU of label was detected in each reading window. For each reading window, the reaction kinetics were measured in 0.2 second intervals.

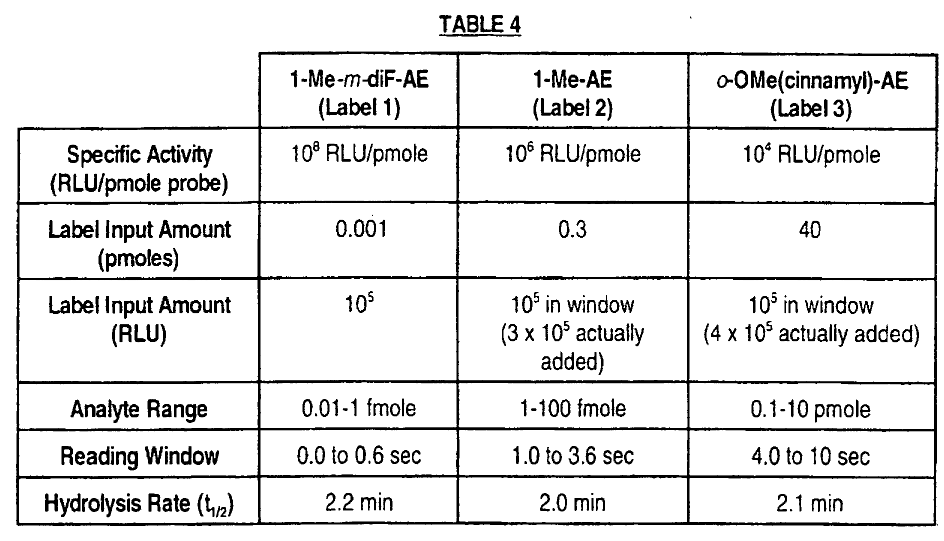

The hydrolysis rate mentioned in Table 4 is important only to the homogeneous assay method illustrated in this example, and is not crucial to the general methods or compositions of the present invention. Table 5 presents the calculated signals for each label in its respective reading window for different amounts of target, based on the assumption that the labels are completely distinguishable.

**TABLE 5**

| **Target (pmole)** | **Label 1 (RLU)** | **Label 2 (RLU)** | **Label 3 (RLU)** |
|---|---|---|---|
| 0.00001 | 1000 | 10 | 0.1 |
| 0.00004 | 4000 | 40 | 0.4 |
| **0.00007** | 7000 | 70 | 0.7 |
| **0.0001** | 10⁴ | 100 | 1.0 |
| **0.0004** | 4 x 10⁴ | 400 | 4.0 |
| **0.0007** | 7 x 10⁴ | 700 | 7.0 |
| **0.001** | 10⁵ | 1000 | 10 |
| **0.004** | 10⁵ | 4000 | 40 |
| **0.007** | 10⁵ | 7000 | 70 |
| **0.01** | 10⁵ | 10⁴ | 100 |
| **0.04** | 10⁵ | 4 x 10⁴ | 400 |
| **0.07** | 10⁵ | 7 x 10⁴ | 700 |
| **0.10** | 10⁵ | 10⁵ | 1000 |
| **0.40** | 10⁵ | 10⁵ | 4000 |
| **0.70** | 10⁵ | 10⁵ | 7000 |
| **1.0** | 10⁵ | 10⁵ | 10⁴ |
| **4.0** | 10⁵ | 10⁵ | 4 x 10⁴ |
| **7.0** | 10⁵ | 10⁵ | 7 x 10⁴ |
| **10.0** | 10⁵ | 10⁵ | 10⁵ |

To verify this hypothetical analysis, each of these three acridinium ester labels was mixed in the proportions indicated above in a solution containing 30 µl of 10 mM lithium succinate (pH 5.0) and 0.1% (w/v) lithium lauryl sulfate, 100 µl of 100 mM lithium succinate (pH 5.0), 8.5% (w/v) lithium lauryl sulfate, 1.5 mM EDTA, 1.5mM EGTA, and 300 µl of 600 mM sodium borate (pH 8.5), 1% (v/v) TRITON® X-100 (octylphenoxy polyethoxyethanol). This solution was placed into a luminometer (LEADER® 50), and detected after injection of 200 µl 0.1% (v/v) H₂O₂ in 1 mM HNO₃, followed by an injection of 200 µl of 1.5 N NaOH. Light emission was measured at 0.2 second intervals after initiation of the chemiluminescent reaction. The reading windows were the same as in Table 4. Table 6 presents the experimental results obtained.

**TABLE 6**

| **Target (pmole)** | **Label 1 (RLU)** | **Label 2 (RLU)** | **Label 3 (RLU)** |
|---|---|---|---|
| 0.00001 | 1,080 | 54 | 63 |
| 0.00004 | 4,601 | 188 | 86 |
| 0.00007 | 7,697 | 311 | 198 |
| 0.0001 | 10,197 | 380 | 234 |
| 0.0004 | 40,959 | 1,308 | 704 |
| 0.0007 | 66,174 | 2,295 | 1,166 |
| 0.001 | 90,979 | 3,117 | 1,810 |
| 0.004 | 93,129 | 8,353 | 2,156 |
| 0.007 | 97,467 | 12,604 | 2.360 |
| 0.01 | 97,010 | 16,285 | 2,516 |
| 0.04 | 144,034 | 61,496 | 5,653 |
| 0.07 | 184,345 | 102,891 | 8,022 |
| 0.10 | 179,248 | 142,326 | 10,524 |
| 0.40 | 180,289 | 146,349 | 14,517 |
| 0.70 | 180,901 | 153,413 | 19,049 |
| 1.0 | 177,833 | 154,494 | 21,023 |
| 4.0 | 197,599 | 232,885 | 59,264 |
| 7.0 | 202,855 | 292,157 | 90,589 |
| 10.0 | 220,880 | 346,219 | 121,487 |

The results of Table 6 show that, in comparison, the data actually obtained correspond well with the theoretical values of Table 5 when the labels were mixed and detected in separate detection windows. As predicted, Label 1 gave a linear response until it reached saturation, at a level of about 10⁵ RLU. Similarly, Label 2 permits detection to a theoretical target amount of 0.1 pmoles, because the signal given off by Label 2 also reached a plateau at about 10⁵ RLU. Lastly, the signal emitted from Label 3 rose above background at a theoretical target level of between 0.01 and 0.04 pmoles and increased proportionally to the amount of target to the maximum theoretical target amount of 10 pmoles.

The experiments in this example were conducted in the absence of target and without each label being joined to a oligonucleotide probe. However, those skilled in the art will recognize that the present results are predicted to be similar to those obtained when each label is joined to a probe at the indicated specific activity in an actual assay of target analyte across the ranges from 0.01 fmoles to 10 pmoles, so long as the probes are each targeted to a different region of the target analyte.

### EXAMPLE 3

### Extending the Dynamic Range of More Than One Analyte

The present invention may be used in conjunction with four or more separately detectable labels to extend the dynamic range for the detection of two or more analytes in a multiple analyte assay system. Labels may be of any type, for example, radioactive, fluorescent, chemiluminescent, chromogeneic, and enzyme- or substrate-linked labels. Examples of multiple analyte assay systems have been described in detail previously (e.g.,US Pat. No. 5,656, 207 to Woodhead et al., and US Pat. No. 5,658,737 to Nelson et al.). Although these references describe particular multiple analyte assay systems, as the previous discussion makes clear, the present invention may be used predictably in a variety of known assay systems.

In an assay to detect the presence of multiple analytes, at least two labeled probes specific for different target regions of an analyte are used to detect each different analyte. The dynamic range of the assay for the detection of each analyte is extended by using at least two labeled probes targeted to different regions of each said analyte. As in Example 2, each analyte is detected using at least two such probes preferably labeled with distinguishable labels. Preferably probes targeted to the same analyte are labeled at different specific activities, with the amount of each probe corresponding to the upper limit of the range of analyte amount which that probe is intended to detect.

In this example, four probes are used (designated Probes 1, 2, 3 and 4) that bind to two different analytes (Analyte 1 and Analyte 2). Probes 1 and 2 specifically bind to Analyte 1 at distinct target regions. Probe 1 is labeled using standard procedures with a fluorescent label, 5-carboxy-fluorescein (excitation at 492 nm and emission at 518 nm) to a specific activity of 10⁶ relative fluorescent units ("RFU") per pmol of probe. Probe 2 is similarly labeled with a different fluorescent label, 6-carboxy rhodamine (excitation at 518 nm and emission at 543 nm) and then mixed with unlabeled Probe 2 to achieve a final specific activity of 10⁴ RFU/pmol. Probes 3 and 4 specifically bind to Analyte 2 at distinct target regions. Probe 3 is labeled with a fluorescent label, 5-(and-6-)-carboxytetramethylrhodamine (excitation at 540 nm and emission at 565 nm) to a specific activity of 10⁶ RFU/pmol. Probe 4 is labeled with sulforhodamine 101 acid chloride ("Texas Red®"; excitation at 587 nm and emission at 602 nm) and then mixed with unlabeled Probe 4 to achieve a final specific activity of 10⁴ RFU/pmol. The four labels used in this example are independently distinguishable based on the different wavelengths of their fluorescence emissions. Those skilled in the art will appreciate that other types of labels could readily be substituted for any of these, so long as distinguishable signals are achieved in the combination of labeled probes.

In this assay, the probes are mixed together as follows:

| **Probe** | **Amount (pmol)** | **Total RFU at emission wavelength** |
|---|---|---|
| Probe 1 | 0.1 | 10⁵ at 518 nm |
| Probe 2 | 10 | 10⁵ at 543 nm |
| Probe 3 | 0.1 | 10⁵ at 565 nm |
| Probe 4 | 10 | 10⁵ at 602 nm |

The mixtures are contacted with samples containing different amounts of one or both of the analytes (i.e., Analyte 1, Analyte 2 or Analytes 1 + 2) under conditions that favor binding of the probes to their respective analyte target regions. After binding, a step of distinguishing bound from unbound probe is performed (e.g., washing), using methods that can readily be determined by one skilled in the art. Then, the fluorescence signal of each of the bound labeled probes is detected at the appropriate wavelength.

Assuming complete binding of each analyte and its specific probes, the signals detectable for each of the labeled probes are shown in Table 7 for different amounts of analyte (pmol) in the sample. For each probe, background is 0.1% of the total RFU added to the mixture (i.e., 10² RFU per label).

**Table 7**

| **Analyte & Amount** **(pmol)** | **Probe 1 RFU** **(at 518 nm)** | **Probe 2 RFU** **(at 543 nm)** | **Probe 3 RFU** **(at 565 nm)** | **Probe 4 RFU** **(at 602 nm)** |
|---|---|---|---|---|
| Analyte 1 | 10² | 10² | 10² | 10² |
| 0.0001 | | | | |
| Analyte 1 | 10³ | 10² | 10² | 10² |
| 0.001 | | | | |
| Analyte 1 | 10⁴ | 10² | 10² | 10² |
| 0.01 | | | | |
| Analyte 1 | 10⁵ | 10³ | 10² | 10² |
| 0.1 | | | | |
| Analyte 1 | 10⁵ | 10⁴ | 10² | 10² |
| 1.0 | | | | |
| Analyte 1 | 10⁵ | 10⁵ | 10² | 10² |
| 10 | | | | |
| Analyte 2 | 10² | 10² | 10² | 10² |
| 0.0001 | | | | |
| Analyte 2 | 10² | 10² | 10³ | 10² |
| 0.001 | | | | |
| Analyte 2 | 10² | 10² | 10⁴ | 10² |
| 0.01 | | | | |
| Analyte 2 | 10² | 10² | 10⁵ | 10³ |
| 0.1 | | | | |
| Analyte 2 | 10² | 10² | 10⁵ | 10⁴ |
| 1.0 | | | | |
| Analyte 2 | 10² | 10² | 10⁵ | 10⁵ |
| 10 | | | | |
| Analytes 1+ 2 | 10² | 10² | 10² | 10² |
| 0.0001 of each | | | | |
| Analytes 1+ 2 | 10³ | 10² | 10³ | 10² |
| 0.001 of each | | | | |
| Analytes 1+ 2 | 10⁴ | 10² | 10⁴ | 10² |
| 0.01 of each | | | | |
| Analytes 1+ 2 | 10⁵ | 10³ | 10⁵ | 10³ |
| 0.1 of each | | | | |
| Analytes 1+ 2 | 10⁵ | 10⁴ | 10⁵ | 10⁴ |
| 1.0 of each | | | | |
| Analytes 1+ 2 | 10⁵ | 10⁵ | 10⁵ | 10⁵ |
| 10 of each | | | | |

In this example, each probe individually can detect its respective analyte over 3 logs of concentration, and the combination of probes specific for one analyte as described above can detect the analyte over 5 logs of concentration. Furthermore, as shown by the RFU in Table 7, the combination of four probes detects and quantitates both analytes simultaneously and independently over an extended dynamic range of concentrations. Thus, this type of assay format allows the separate detection and/or quantification of two or more analytes, with each analyte being accurately measured over a wide concentration range.

Although this example describes an assay in which all of the probe labels are fluorescent labels, it will be understood that other labels or combinations of types of labels could equally be used. In one example, labels on Probes 1 and 2 are 5-carboxy-fluorescein and 6-carboxy rhodamine, respectively, and labels on Probes 3 and 4 are ³H and ³²P, respectively. After performing the assay steps described above, fluorescence signals are measured at 518 nm and 543 nm and then the different radioactive labels are detected and distinguished using any of a variety of well known methods. In another example, labels on Probes 1 and 2 are 5-carboxy-fluorescein and 6-carboxy rhodamine, respectively, and labels on Probes 3 and 4 are acridinium ester derivatives such as o-F-AE and 2-Me-AE, respectively. After performing the assay steps described above, fluorescence signals are measured at 518 nm and 543 nm, and then the chemiluminescence is measured using well known methods that distinguish the signals from the two different acridinium ester labels.

In another example using different distinguishable labels, labels on Probes 1 and 2 are alkaline phosphatase and horseradish peroxidase, respectively, and the labels on Probes 3 and 4 are 5-(and-6-)-carboxytetramethylrhodamine and Texas Red@, respectively. After performing the assay steps described above, appropriate enzyme substrates are added and allowed to react with the labels of analyte-bound Probes 1 and 2 (e.g., providing p-nitrophenyl phosphate for alkaline phosphatase and measuring the soluble end product by measuring absorbance at 405 nm; and providing o-phenylenediamine for horseradish peroxidase and measuring the soluble end product by measuring absorbance at 450 nm). After the bound enzyme labels have been detected, the fluorescence signals of bound Probes 3 and 4 are measured at 565 nm and 602 nm. For each of these examples, one skilled in the art can readily determine adjustments of specific activity needed for each probe to allow detection of the probes as described above and demonstrated by the information in Table 7.

Those skillled in the art will appreciate that different combinations of labels (e.g., radionuclides, chemiluminescence labels, fluorescence labels), as described above for assays using four labeled probes, could readily be used in assays using two labeled probes. That is, for detection of a single analyte of an extended dynamic range, one would perform the above described assay using any combination of two distinguishably-labeled probes that specifically recognize different targets of the analyte (e.g., Probes 1 and 2 alone).

### EXAMPLE 4

### Demonstration of Extended Dynamic Range Using Different Oligonucleotide Probes

This example demonstrates the ability of one embodiment of the present invention to specifically detect analyte across a broad range of analyte amount or concentration. Two different probes bearing distinguishable labels were used to detect an analyte in samples, where the analyte concentration varied over a range of five orders of magnitude. Initially, the individual probes were characterized independently, and then the probes were combined in a single analyte-detection assay.

A synthetic 48-base RNA oligomer was used as the target analyte. Two different oligonucleotide probes complementary to non-overlapping, unique areas of the target RNA (Probe 1 of 22 nucleotides, and Probe 2 of 18 nucleotides) were labeled with different chemiluminescent labels. Probe 1 was labeled with an N-acridinium phenyl ester having a fluorine substitution at the ortho position of the phenyl ring (i.e., *o*-F-AE), and Probe 2 was labeled with an N-acridinium phenyl ester having a methyl group at the 2' position of the acridinium ring (i.e., 2-Me-AE). An unsubstituted acridinium ester containing a NHS linker at the 4 position of the phenyl moiety is 4-(2-succinimidyloxycarbonyl ethyl) phenyl-10-methylacridinium 9-carboxylate fluorosulfonate. Both Probes 1 and 2 contained modified sugar moieties wherein the 2' position of the ring was substituted with a methoxy group rather than -H (deoxyribose) or -OH (ribose), but use of modified oligonucleotides is not critical to the methods of the present invention, which can be performed with unmodified oligonucleotides as well.

A chemiluminescent reaction employing the *o*-F-AE label has sufficiently fast reaction kinetics compared to those of the 2-Me-AE label that the signals from the two compounds following the reaction initiating chemiluminescence can be distinguished from each other by monitoring the resulting light emission over a time penod at regular intervals. The respective signals obtained from the two labels can be even more precisely quantified by employing a simple reiterative mathematical technique. These methods and representative labels have been previously described in greater detail in US Pat. No. 5,656,207 to Woodhead et al., and US Pat. No. 5,658,737 to Nelson et al.

The oligonucleotides of Probes 1 and 2 were linked to the AE labels through the use of a linker arm, as shown in FIGS. 1A to 1C, attached to the phenyl ring. The linker was joined to the oligonucleotide via a non-nucleotide linker, which was incorporated into the oligonucleotide chain using methods previously described in detail in US Pat. No. 5,656,744 to Arnold, et al. In this method the linker arm moiety to which the label will be attached is placed at a predetermined position within the oligonucleotide during synthesis. Oligonucleotides were synthesized using well-known solid-phase synthesis methods (e.g., as described in Brown & Brown, "Modern Machine-Aided Methods of Oligodeoxyribonucleotide Synthesis" in *Oligonucleotides and Analogues, A Practiced Approach* (1991)). Acridinium ester derivatives may be joined to the linker arm of the probe using techniques well known in the art, but preferred methods have been previously described in Nelson et al., "Detection of Acridinium Esters by Chemiluminescence" in *Non-lsotopic Probe Techniques* (Academic Press 1992), and US Pat. No. 5,656,744 to Arnold et al.

In a preferred method, an N-hydroxysuccinimide ("NHS") ester of acridinium (e.g., 4-(2-succinimidyloxycarbonyl ethyl) phenyl-10-methylacridinium 9-carboxylate fluorosulfonate) is synthesized substantially as described in Weeks et al., 1983, *Clin. Chem* 29: 1474-1478, and US Pat. No. 5,658,737 to Nelson et al. Reaction of the primary amine of the linker arm:hybridization probe conjugate with the selected NHS-acridinium ester is performed as follows. The oligonucleotide hybridization probe:linker arm conjugate synthesized as described above is vacuum-dried in a drying centrifuge (e.g., Savant SPEED-VAC™), and then dissolved in 8 µl of 0.125 M HEPES buffer (pH 8.0) in 50% (v/v) DMSO. To this, 2 µl of a 25 mM solution of the desired NHS-acridinium ester is added, mixed and incubated at 37 °C for 20 min. Then, 3 µl of 25 mM NHS-acridinium ester in DMSO is added to the solution, mixed gently, and 2 µl of 0.1 M HEPES buffer (pH 8.0) is added, mixed, and incubated at 37°C for 20 min. more. The reaction is quenched by gently mixing with 5 µl of 0.125 M lysine in 0.1 M HEPES buffer (pH 8.0) in DMSO.

The labeled oligonucleotide is recovered by the addition of 30 µl of 3 M sodium acetate buffer (pH 5.0), 245 µl of water, and 5 µl of 40 mg/ml glycogen, and then adding 640 µl of chilled 100% ethanol, mixing and incubating on dry ice for 5 to 10 min. The precipitated labeled nucleic acids are sedimented in a refrigerated microcentrifuge at 15,000 rpm using a standard rotor head. The supernatant is aspirated off, and the pellet is redissolved in 20 µl of 0.1 M sodium acetate (pH 5.0) containing 0.1% (w/v) sodium dodecyl sulfate (SDS).

Probes 1 and 2, conjugated to AE labels substantially as described above, were used at different specific activities. Probe 1 was labeled with *o*-F-AE at a specific activity of about 7 x 10⁷ RLU/pmole. Probe 2 was labeled with 2-Me-AE at a specific activity of about 1 x 10⁸ RLU/pmole and then mixed with unlabeled Probe 2 (as described below) to yield a lower specific activity. This resulted in about a 100-fold difference between the detectable signals of Probe 1 and Probe 2.

To test the two separate probe and label combinations in an assay, 11 fmoles of labeled Probe 1 were hybridized to various amounts (0.00, 0.01, 0.02, 0.05, 0.20, 0.50, 2, 5, 20, 50, 200, 500, 2000, and 5000 fmoles) of the target RNA. At the same time, 5 fmoles labeled Probe 2 plus 15 pmoles unlabeled Probe 2 were hybridized in separate reaction mixtures to the same amounts of target. In addition to probe and target RNA, each hybridization reaction of 100 µl contained 100 mM lithium succinate (pH 5.0), 8.5% (w/v) lithium lauryl sulfate, 1.5 mM EDTA, and 1.5 mM EGTA. The reaction mixtures were incubated at 50°C for 50 min, and then 300 µl of 150 mM Na₂B₄O₇ (pH 8.6) containing 1% (v/v) TRITON® X-100 were added to each reaction, and the mixtures were incubated at 50°C for 11 min. The reaction mixtures were then placed into a luminometer (LEADER® 50) and a chemiluminescent reaction was initiated in each mixture upon the injection of 200 µl of 0.1% (v/v) H₂O₂ and 1 mM HNO₃, followed by injection of 200 µl of 1.5 N NaOH. Chemiluminescence was measured at a wavelength range from 300 to 650 nm for 2 sec following the second injection.

The results are shown in Table 8 below and graphically illustrated on a log-log scale in FIGS. 3A and 3B

**TABLE 8**

| **Analyte Assayed and Detected in Individual Reaction Mixtures** | | |
|---|---|---|
| **Target RNA (fmoles)** | **RLU Output of Probe 1 (*o*-F-AE)** | **RLU Output of Probe 2 (2-Me-AE)** |
| 0.00 | 53 | 0 |
| 0.01 | 428 | 0 |
| 0.02 | 912 | 0 |
| 0.05 | 2,209 | 0 |
| 0.2 | 9,181 | 0 |
| 0.5 | 22,968 | 0 |
| 2.0 | 82,545 | 0 |
| 5.0 | 177,543 | 903 |
| 20 | 264,501 | 1,673 |
| 50 | 311,928 | 3,979 |
| 200 | 324,618 | 15,304 |
| 500 | 322,676 | 39,502 |
| 2000 | 351,596 | 158,033 |
| 5000 | 330,214 | 275,699 |

As shown by the results of Table 8 and FIG. 3A, when assayed in separate reactions, the o-F-AE label of Probe 1 emitted an increasing amount of light with increasing amount of analyte in the reaction. The response was linear from about 0.01 fmoles to about 5 fmoles of the RNA analyte and in excess of 5 fmoles of analyte, the reaction began to plateau. Similarly, the 2-Me-AE label of Probe 2 emitted more light when the amount of analyte present in the reaction was increased, but the detectable signal of the 2-Me-AE label of Probe 2 was about 100-fold lower than that of the o-F-AE label of Probe 1. Thus, light emitted by target-bound Probe 2 only became detectable when more analyte was present, and a linear response (see FIG. 3B) was seen when analyte was present in a range of from about 20 fmoles to about 2,000 to 5,000 fmoles. From these experiments, standard curves were generated for each of the two labels, present at different specific activities.

Next, both labeled Probe 1 and labeled Probe 2, as described above, were combined in individual hybridization mixtures, each containing different amounts of the RNA analyte. In each reaction, the detectable signal of Probe 2 was about 100-fold less than the detectable signal of Probe 1. Eleven fmoles *o*-F-AE labeled Probe 1 and 5 fmoles 2-Me-AE labeled Probe 2 were combined with 15 pmoles of unlabeled Probe 2 in 100 µl reactions containing a hybridization buffer (100 mM lithium succinate, pH 5.0, 8.5% (w/v) lithium lauryl sulfate, 1.5 mM EDTA, and 1.5 mM EGTA) and different amounts of RNA target, as described in Example 4. Each reaction mixture was incubated at 50°C for 50 min; then, 300 µl of 150 mM Na₂B₄O₇ ( pH 8.6) containing 1% (v/v) TRITON® X-100 were added to each reaction which were incubated at 50°C for 11 min. Then, the reactions were placed into a luminometer (LEADER® 50), and a chemiluminescent reaction was initiated by injecting into each reaction 200 µl of 0.1% (v/v) H₂O₂ and 1 mM HNO₃, followed by 200 µl of 1.5 N NaOH. Chemiluminescence was then measured for 2 sec, with light collected over 40 millisecond intervals during this period.

The signal emitted by each label was resolved from the signal generated by the mixture of labels as follows. The raw data were subjected to a reiterative mathematical calculation, as described in detail previously (see Nelson, et al., US Pat. No. 5,658,737, at col. 22, lines 37-65) and briefly as follows, using the sum of the light emitted during two interval ranges within the time period (intervals 1 to 5 and intervals 30 to 50, abbreviated as "RLU₁₋₅" and "RLU₃₀₋₅₀", respectively). The intervals are referred to by the number of the 40-millisecond intervals following initiation of the chemiluminescent reaction.

Samples containing only one labeled probe were used as standards for data analysis. For each standard, the ratio between the sum of the RLU values obtained in the first time interval and the sum of the RLU values obtained in the second time interval was determined (Σ RLU₃₀₋₅₀/Σ RLU₁₋₅). For the reaction mixtures containing both labeled probes, the sum of the RLU values in intervals 30 to 50 (Σ RLU₃₀₋₅₀) was divided by the ratio obtained for the 2-Me-AE standard. This results in the calculated amount of RLU contributed in intervals 1 to 5 by the 2-Me-AE-labeled probe. This amount, subtracted from the total RLU in intervals 1 to 5, gives the amount of RLU contributed in these intervals by *o*-F-AE. The latter number, when multiplied by the standard ratio for the two time periods obtained for *o*-F-AE, yields the RLU within the intervals 30 to 50 which were contributed by the *o*-F-AE-labeled probe. This figure is subtracted from the total RLU in intervals 30 to 50, to obtain a corrected value for the RLU contributed by 2-Me-AE in this interval. This number was used to repeat the calculation described above until the RLU contribution by *o*-F-AE in intervals 30 to 50 did not change within the chosen number of significant figures. Calculated amounts of RLU ("RLU Output") for Probes 1 and 2 are shown in Table 9.

**TABLE 9**

| **Analyte Assayed and Detected in Single Reaction Mixtures** | | |
|---|---|---|
| **fmoles Target RNA** | **RLU Output of Probe 1 (o-F-AE)** | **RLU Output of Probe 2 (2-Me-AE)** |
| 0.00 | 59 | 63 |
| 0.01 | 573 | 31 |
| 0.02 | 1,233 | 31 |
| 0.05 | 2,893 | 0 |
| 0.2 | 11,068 | 0 |
| 0.5 | 27,139 | 31 |
| 2.0 | 93,535 | 189 |
| 5.0 | 204,984 | 600 |
| 20.0 | 271,543 | 2,277 |
| 50.0 | 315,697 | 4,428 |
| 200.0 | 314,792 | 15,974 |
| 500.0 | 301,076 | 39,036 |
| 2000.0 | 321,450 | 153,234 |
| 5000.0 | 328,863 | 279,105 |

These data demonstrate the ability of the methods of the present invention to extend the dynamic range for detection of an analyte. As shown in FIG. 4, the graphic representation of the results of this experiment closely resemble a composite of the results graphed in FIGS. 3A and 3B. The method illustrated here, in which probes are directed to different target regions of the same analyte, permits the detection and/or quantification of the analyte over at least 6 logs of possible target concentrations in a single-vessel assay. Therefore, this assay format could be used to detect or measure an analyte in a sample within this extended range of analyte concentrations without prior knowledge of the analyte concentration in the sample.

FIG. 4 also shows that the methods of the present invention permit detection, within the luminometer reporting range, of at least 6 logs of analyte concentrations by using independently detectable labels at different specific activities. Even further expansion of the dynamic range of the assay is possible, for example, by using a greater number of probes linked to independently detectable labels but within the luminometer reporting range of any selected instrument. Thus, an assay can be custom designed to utilize the most accurate reporting range of the instrument or detection method used.

### EXAMPLE 5

### Extended Dynamic Range Using Different Probes Labeled With the Same Detectable Label

This example shows that two probes that specifically bind to different target regions of the same analyte, when labeled with the same detectable label but at different specific activities, can be used to detect an extended range of analyte concentrations.

The probes used in this example are synthetic DNA oligomers having a 2-methoxy backbone, and designated Probe 3 (a 22-mer) and Probe 4 (a 19-mer). Both probes contain nucleotide base sequences that specifically bind to the same analyte but to separate non-overlapping target regions of the analyte. The analyte was a synthetic RNA oligomer consisting of 48 bases. Both Probes 3 and 4 were labeled with standard AE substantially as described in Example 4. Labeled Probe 4 was mixed with unlabeled Probe 4 to achieve a lower specific activity relative to the specific activity of Probe 3. The probe having the higher specific activity (Probe 3) was used to detect the lower range of analyte concentrations, and the probe with the lower specific activity (Probe 4) was used to detect the higher range of analyte concentrations.

For each probe, a series of hybridization and chemiluminescence detection reactions were performed substantially as described in Example 4, using the RNA analyte combined with either Probe 3 without Probe 4; Probe 4 without Probe 3; or a combination of Probes 3 and 4. The amount of analyte added to the reactions was: 0.1, 1.0, 10, 100, 1,000, 10,000 and 100,000 fmol/reaction. Each reaction condition was performed in triplicate and the average (mean) RLU results were calculated from the triplicate samples. Triplicate tubes containing no analyte were used to determine background RLU, which were averaged and subtracted from the average of RLU results for each experimental test.

The reactions were set up substantially as described in Example 4, using the following amounts of probe in 100 µl reaction mixtures: 50 fmol of labeled Probe 3 were added to those reactions containing Probe 3 alone or combined with Probe 4; 50 fmol of labeled Probe 4 plus 20 pmol of unlabeled Probe 4 were added to those reactions containing Probe 4 alone or combined with Probe 3. The hybridization reactions were incubated at 59 °C for 30 min, and then 300 µl of 150 mM Na2B4O7 (pH 8.7) containing 1% Triton®X-100 was added to each tube which was then incubated at 59°C for 9 min. This was followed by initiating and detecting the chemiluminescent reaction substantially as described in Example 4. Table 10 presents the results of these experiments.

**TABLE 10**

| **fmoles Target RNA** | **Mean RLU Detected Probe 3 Alone** | **Mean RLU Detected Probe 4 Alone** | **Mean RLU Detected Probes 3 and 4** |
|---|---|---|---|
| 0.1 | 3,630 | Not Tested | 596 |
| 1.0 | 36,758 | Not Tested | 38,250 |
| 10 | 379,672 | 16,270 | 405,427 |
| 100 | 2,054,816 | 122,222 | 2,120,323 |
| 1,000 | 2,047,840 | 1,474,208 | 3,618,762 |
| 10,000 | Not Tested | 10,217,034 | 11,717,176 |
| 100,000 | Not Tested | 10,507,565 | 12,646,943 |

The results of Table 10 are graphically shown in FIGS. 5A to 5C, using log-log scale graphs of the RLU detected as a function of the analyte present in the sample. Referring to FIG. 5A, the results obtained with Probe 3 alone hybridized to its target are shown, clearly showing a linear response over about a three log range of analyte concentrations (0.1 to 100 fmol). Referring to FIG. 5B, the results obtained with Probe 4 alone hybridized to its target are shown, clearly showing a linear response over about a three log range of analyte concentrations (10 to 10,000 fmol). Referring to FIG. 5C, when both probes were present in the hybridization reactions, the data points fall on two distinct lines. FIG. 5C shows that two probes that produce the same detectable signal and specifically bind to different target regions on the same analyte can be used in a single reaction to produce detectable signal that can be correlated with the concentration of analyte present in a sample over a range of at least 5 orders of magnitude. Thus, a combination of different probes labeled with the same label but at different specific activities and used in a format as illustrated in this example can detect the presence of analyte over an extended range of analyte concentrations than would be detected with either probe alone. Moreover, the combination of probes can be used to quantify the amount of analyte present in a sample over this extended concentration range.

The preceding examples are intended to illustrate rather than limit the present invention, which is defined by the claims that follow and legal equivalents thereof.

## Claims

1. A method of detecting an analyte over a range of concentrations in a single sample, comprising the steps of:
providing a probe reagent comprising at least two different labeled probes,
wherein each labeled probe comprises a target binding moiety and a detectable label,
wherein the target binding moiety of each labeled probe binds specifically to a target region that differs from the target region bound by another labeled probe in the probe reagent, and
wherein each labeled probe is present in an amount capable of detecting a range of analyte concentrations that differs from the range of analyte concentrations detectable by another probe in the reagent, thereby allowing the probe reagent to detect an extended range of analyte concentrations that is greater than the range of analyte concentrations detectable by a single labeled probe in the probe reagent;
contacting in a single vessel the probe reagent with a sample containing the analyte,
wherein the probe reagent contains a first probe present in at least a 10-fold molar excess relative to a second probe;
incubating the sample and the probe reagent under conditions favoring specific binding of the target binding moiety of each labeled probe with its target region of the analyte, thereby producing a specific binding complex comprising the analyte and at least one labeled probe; and
detecting the presence of the specific binding complex by detecting a signal from the label indicating the presence of the at least one probe in the specific binding complex, thereby indicating the presence of the analyte in the sample within the range of analyte concentrations detectable by the probe in the specific binding complex.

2. The method of claim 1, wherein the contacting and incubating steps are performed in a single vessel.

3. The method of claim 1 or 2, wherein the detecting step measures a signal from the detectable label that is characteristic of kinetics of a reaction.

4. The method of any one of claims 1 to 3, wherein the providing step uses a probe reagent in which specific activity of one labeled probe differs from specific activity of another labeled probe in the probe reagent.

5. The method of any one of claims 1 to 4, wherein the providing step uses a probe reagent wherein at least one labeled probe has a detectable label that is a ligand capable of specifically binding a signal-producing binding partner.

6. The method of any one of claims 1 to 4, wherein the providing step uses a probe reagent wherein at least one labeled probe has a detectable label that is a direct label, and the detecting step measures a signal resulting from the direct label.

7. The method of any one of claims 1 to 4, wherein the providing step uses a probe reagent wherein at least one labeled probe has a detectable label that is a fluorescent label, luminescent label, radioactive label, chromophore, an enzyme, or an enzyme substrate.

8. The method of any one of claims 1 to 6, wherein the providing step uses a probe reagent wherein at least one labeled probe has a detectable label that is a chemiluminescent compound

9. The method of any one of claims 1 to 8, wherein the contacting step uses the probe reagent wherein the first labeled probe is present in at least a 100-fold, 1,000-fold or 10,000-fold molar excess relative to the second labeled probe.

10. The method of any one of claims 1 to 9, wherein the probe reagent comprises the first labeled probe having a detectable label that is a first chemiluminescent compound and the second labeled probe having a detectable label that is a second chemiluminescent compound, wherein the first chemiluminescent compound and the second chemiluminescent compound are separately distinguishable labels.

11. The method of claim 10, wherein the first chemiluminescent compound is selected from the group consisting of standard acridinium ester (AE), naphthyl-AE, *ortho*-AE, 1- or 3-methyl-AE, 2-methyl-AE, 2,7-dimethyl-AE, 4,5-dimethyl-AE, *ortho*-dibromo-AE, *ortho*-dimethyl-AE, *meta*-dimethyl-AE, *ortho*-methoxy-AE, *ortho*-methoxy(cinnamyl)-AE, *ortho*-methyl-AE, *ortho*-fluoro-AE, 1- or 3-methyl-*ortho*-fluoro-AE and 1- or 3-methyl-*meta*-difluoro-AE; and
wherein the second chemiluminescent compound is selected from the group consisting of standard acridinium ester (AE), naphthyl-AE, *ortho*-AE, 1- or 3-methyl-AE, 2-methyl-AE, 2,7-dimethyl-AE, 4,5-dimethyl-AE, *ortho*-dibromo-AE, *ortho*-dimethyl-AE, *meta*-dimethyl-AE, *ortho*-methoxy-AE, *ortho*-methoxy(cinnamyl)-AE, *ortho*-methyl-AE, *ortho*-fluoro-AE, 1- or 3-methyl-*ortho*-fluoro-AE and 1- or 3-methyl-*meta*-difluoro-AE,
provided that the first chemiluminescent compound is different and distinguishable from the second chemiluminescent compound.

12. The method of claim 11, wherein the first chemiluminescent compound is ortho-fluoro-AE, and the second chemiluminescent compound is 2-methyl-AE.

13. The method of claim 10, wherein the probe reagent further comprises a third labeled probe having a separately distinguishable label that is a third chemiluminescent compound.

14. The method of claim 13, wherein the first chemiluminescent compound is selected from the group consisting of standard acridinium ester (AE), naphthyl-AE, *ortho*-AE, 1- or 3-methyl-AE, 2-methyl-AE, 2,7-dimethyl-AE, 4,5-dimethyl-AE, *ortho*-dibromo-AE, *ortho*-dimethyl-AE, *meta*-dimethyl-AE, *ortho*-methoxy-AE, *ortho*-methoxy(cinnamyl)-AE, *ortho*-methyl-AE, *ortho*-fluoro-AE, 1- or 3-methyl-*ortho*-fluoro-AE and 1- or 3-methyl-*meta*-difluoro-AE;
wherein the second chemiluminescent compound is selected from the group consisting of standard acridinium ester (AE), naphthyl-AE, *ortho*-AE, 1- or 3-methyl-AE, 2-methyl-AE, 2,7-dimethyl-AE, 4,5-dimethyl-AE, *ortho*-dibromo-AE, *ortho*-dimethyl-AE, *meta*-dimethyl-AE, *ortho*-methoxy-AE, *ortho*-methoxy(cinnamyl)-AE, *ortho*-methyl-AE, *ortho*-fluoro-AE, 1- or 3-methyl-*ortho*-fluoro-AE and 1- or 3-methyl-*meta*-difluoro-AE; and
wherein the third chemiluminescent compound is selected from the group consisting of standard acridinium ester (AE), naphthyl-AE, *ortho*-AE, 1- or 3-methyl-AE, 2-methyl-AE, 2,7-dimethyl-AE, 4,5-dimethyl-AE, *ortho*-dibromo-AE, *ortho*-dimethyl-AE, *meta*-dimethyl-AE, *ortho*-methoxy-AE, *ortho*-methoxy(cinnamyl)-AE, *ortho*-methyl-AE, *ortho*-fluoro-AE, 1- or 3-methyl-*ortho*-fluoro-AE and 1-or 3-methyl-*meta*-difluoro-AE,
provided that the first, second and third chemiluminescent compounds differ and are distinguishable from each other.

15. The method of claim 14, wherein the first chemiluminescent compound is 1- or 3-methyl-*meta*-difluoro-AE, the second chemiluminescent compound is 1- or 3-methyl-AE, and the third chemiluminescent compound is *ortho*-methoxy(cinnamyl)-AE.

16. The method of any one of claims 1 to 15, wherein the contacting step includes an analyte that is a nucleic acid, protein, lipid, carbohydrate or a compound that is a combination thereof.

17. The method of any one of claims 1 to 16, wherein the contacting step includes an analyte that is a DNA or RNA.

18. The method of any one of claims 1 to 17, wherein the providing step uses a probe reagent wherein at least one labeled probe is a nucleic acid, protein, lipid, carbohydrate or compound that is a combination thereof.

19. The method of any one of claims 1 to 18, wherein the providing step uses a probe reagent wherein at least one labeled probe comprises a DNA, RNA, DNA analog, RNA analog, or a combination thereof.

20. A probe reagent for use in the method of any one of claims 1 to 19, comprising at least two different labeled probes, each labeled probe comprising a target binding moiety and a detectable label,
wherein the target binding moiety of each labeled probe binds specifically to a target region that differs from the target region bound by another labeled probe in the probe reagent, and
wherein each labeled probe is present in an amount capable of detecting a range of analyte concentrations that differs from a range of analyte concentrations detectable by another probe in the reagent, and
wherein the probe reagent contains a first probe present in at least a 10-fold molar excess relative to a second probe, thereby allowing the probe reagent to detect the analyte over an extended range of analyte concentrations that is greater than the range of analyte concentrations detectable by a single probe in the probe reagent.

21. The probe reagent of claim 20, wherein the probe reagent includes a first labeled probe present in an amount that differs by at least a 100-fold molar excess, or at least a 1,000-fold molar excess, or at least a 10,000-fold molar excess relative to a second labeled probe present in the probe reagent.

22. The probe reagent of claim 20 or 21, wherein each labeled probe is distinguishable from each other labeled probe in the probe reagent because of:
different specific activities of the different labeled probes,
a signal resulting from separately distinguishable labels on each of the labeled probes,
or a combination thereof.

23. The probe reagent of any one of claims 20 to 22, wherein each labeled probe has a different specific activity of label compared to another labeled probe in the probe reagent.

24. The probe reagent of claim 23, wherein a labeled probe having a relatively high specific activity is present in a molar amount that is lower than a molar amount of a labeled probe having a relatively low specific activity in the probe reagent.

25. The probe reagent of any one of claims 20 to 24, wherein each labeled probe is a nucleic acid oligonucleotide having a target binding moiety that is a base sequence.

26. The probe reagent of any one of claims 20 to 25, wherein each labeled probe has a separately detectable label that is an acridinium ester derivative.

## Patentansprüche

1. Ein Verfahren zum Detektieren eines Analyten über einen Bereich von Konzentrationen in einer einzelnen Probe, das die Schritte umfasst:
Bereitstellen eines Sondenreagenzes, das mindest zwei unterschiedlich markierte Sonden umfasst,
wobei jede markierte Sonde einen Target-bindenden Rest und einen detektierbaren Marker umfasst;
wobei der Target-bindende Rest jeder markierten Sonde spezifisch an einen Targetbereich bindet, der sich von dem durch eine andere markierte Sonde im Sondenreagenz gebundenen Targetbereich unterscheidet, und
wobei jede markierte Sonde in einer Menge vorhanden ist, die ermöglicht, einen Bereich von Analytkonzentrationen zu detektieren, der sich von dem durch eine andere Sonde im Reagenz detektierbaren Bereich von Analytkonzentrationen unterscheidet, wodurch man das Sondenreagenz einen erweiterten Bereich von Analytkonzentrationen, der größer ist als der Bereich von Analytkonzentratione, der durch eine einzelne markierte Sonde im Sondenreagenz detektierbar ist, detektieren läßt;
In-Kontakt-Bringen des Sondenreagenzes mit einer den Analyten enthaltenden Probe in einem einzigen Behälter,
wobei das Sondenreagenz eine erste Sonde enthält, die mindestens in einem 10-fachen molaren Überschuss relativ zu einer zweiten Sonde vorhanden ist;
Inkubieren der Probe und des Sondenreagenzes unter Bedingungen, die eine spezifische Bindung des Target-bindenden Restes jeder markierten Sonde an ihren Targetbereich am Analyten begünstigt, wodurch ein spezifischer Bindungskomplex gebildet wird, der den Analyten und mindestens eine markierte Sonde umfasst; und
Detektieren der Anwesenheit des spezifischen Bindungskomplexes durch Detektieren eines Signals des Markers als Nachweis für die Anwesenheit der mindestens einen Sonde in dem spezifischen Bindungskomplex, wodurch die Anwesenheit des Analyten in der Probe innerhalb des Bereiches von Analytkonzentrationen, der durch die Sonde in dem spezifischen Bindungskomplex detektierbar ist, nachgewiesen wird.

2. Das Verfahren nach Anspruch 1, wobei der Schritt zum In-Kontakt-Bringen und Inkubieren in einem einzigen Behälter durchgeführt werden.

3. Das Verfahren nach Anspruch 1 oder 2, wobei der Schritt zum Detektieren ein Signal vom detektierbaren Marker, das für die Kinetik einer Reaktion charakteristisch ist, misst.

4. Das Verfahren nach einem der Ansprüche 1 bis 3, wobei der Schritt zum Bereitstellen ein Sondenreagenz verwendet, in dem die spezifische Aktivität einer markierten Sonde sich von der spezifischen Aktivität einer anderen markierten Sonde im Sondenreagenz unterscheidet.

5. Das Verfahren nach einem der Ansprüche 1 bis 4, wobei der Schritt zum Bereitstellen ein Sondenreagenz verwendet, wobei mindestens eine markierte Sonde einen detektierbaren Marker aufweist, der ein Ligand ist, der einen signalerzeugenden Bindungspartners spezifisch binden kann.

6. Das Verfahren nach einem der Ansprüche 1 bis 4, wobei der Schritt zum Bereitstellen ein Sondenreagenz verwendet, wobei mindestens eine markierte Sonde einen detektierbaren Marker aufweist, der ein direkter Marker ist, und der Schritt zum Detektieren ein vom direkten Marker stammendes Signal misst.

7. Das Verfahren nach einem der Ansprüche 1 bis 4, wobei der Schritt zum Bereitstellen ein Sondenreagenz verwendet, wobei mindestens eine markierte Sonde einen detektierbaren Marker aufweist, der ein fluoreszierender Marker, ein lumineszierender Marker, ein radioaktiver Marker, ein Chromophor, ein Enzym oder ein Enzymsubstrat ist.

8. Das Verfahren nach einem der Ansprüche 1 bis 6, wobei der Schritt zum Bereitstellen ein Sondenreagenz verwendet, wobei mindestens eine markierte Sonde einen detektierbaren Marker aufweist, der eine chemilumineszierende Verbindung ist.

9. Das Verfahren nach einem der Ansprüche 1 bis 8, wobei der Schritt zum In-Kontakt-Bringen das Sondenreagenz verwendet, wobei die erste markierte Sonde in einem mindestens 100-fachen, 1.000-fachen oder 10.000-fachen molaren Überschuss relativ zur zweiten markierten Sonde vorhanden ist.

10. Das Verfahren nach einem der Ansprüche 1 bis 9, wobei das Sondenreagenz die erste markierte Sonde mit einem detektierbaren Marker, der eine erste chemilumineszierende Verbindung ist und die zweite markierte Sonde mit einem detektierbaren Marker, der eine zweite chemilumineszierende Verbindung ist, wobei die erste chemilumineszierende Verbindung und die zweite chemilumineszierende Verbindung getrennt voneinander unterscheidbare Marker sind, umfasst.

11. Das Verfahren nach Anspruch 10, wobei die erste chemilumineszierende Verbindung aus der Gruppe ausgewählt wird, die aus üblichen Acridiniumester (AE), Naphthyl-AE, *ortho*-AE, 1- oder 3-Methyl-AE, 2-Methyl-AE, 2,7-Dimethyl-AE, 4,5-Dimethyl-AE, *ortho*-Dibrom-AE, *ortho*-Dimethyl-AE, *meta*-Dimethyl-AE, *ortho*-Methoxy-AE, *ortho*-Methoxy(cinnamyl)-AE, *ortho*-Methyl-AE, *ortho*-Fluor-AE, 1- oder 3-Methyl-*ortho*-Fluor-AE und 1- oder 3-Methyl-*meta*-Difluor-AE besteht; und
wobei die zweite chemilumineszierende Verbindung ausgewählt wird aus der Gruppe, die aus üblichen Acridiniumester (AE), Naphthyl-AE, *ortho*-AE, 1- oder 3-Methyl-AE, 2-Methyl-AE, 2,7-Dimethyl-AE, 4,5-Dimethyl-AE, *ortho*-Dibrom-AE, *ortho*-Dimethyl-AE, *meta*-Dimethyl-AE, *ortho*-Methoxy-AE, *ortho*-Methoxy(cinnamyl)-AE, *ortho*-Methyl-AE, *ortho*-Fluor-AE, 1- oder 3-Methyl-*ortho*-fluor-AE und 1- oder 3-Methyl-*meta*-difluoro-AE besteht,
mit der Massgabe, dass die erste chemilumineszierende Verbindung unterschiedlich und unterscheidbar von der zweiten chemilumineszierenden Verbindung ist.

12. Das Verfahren nach Anspruch 11, wobei die erste chemilumineszierende Verbindung *ortho*-Fluor-AE ist und die zweite chemilumineszierende Verbindung 2-Methyl-AE ist.

13. Das Verfahren nach Anspruch 10, wobei das Sondenreagenz weiter eine dritte markierte Sonde mit einem getrennt unterscheidbaren Marker umfasst, der eine dritte chemilumineszierende Verbindung ist.

14. Das Verfahren nach Anspruch 13, wobei die erste chemilumineszierende Verbindung aus der Gruppe ausgewählt wird, die aus üblichen Acridiniumester (AE), Naphthyl-AE, *ortho*-AE, 1- oder 3-Methyl-AE, 2-Methyl-AE, 2,7-Dimethyl-AE, 4,5-Dimethyl-AE, *ortho*-Dibrom-AE, *ortho*-Dimethyl-AE, *meta*-Dimethyl-AE, *ortho*-Methoxy-AE, *ortho*-Methoxy(cinnamyl)-AE, *ortho*-Methyl-AE, *ortho*-Fluor-AE, 1- oder 3-Methyl-*ortho*-fluor-AE und 1- oder 3-Methyl-*meta*-difluor-AE besteht;
wobei die zweite chemilumineszierende Verbindung aus der Gruppe ausgewählt wird, die aus üblichen Acridiniumester (AE), Naphthyl-AE, *ortho*-AE, 1- oder 3-Methyl-AE, 2-Methyl-AE, 2,7-Dimethyl-AE, 4,5-Dimethyl-AE, *ortho*-Dibrom-AE, *ortho*-Dimethyl-AE, *meta*-Dimethyl-AE, *ortho*-Methoxy-AE, *ortho*-Methoxy(cinnamyl)-AE, *ortho*-Methyl-AE, *ortho*-Fluor-AE, 1-oder 3-Methyl-*ortho*-fluor-AE und 1- oder 3-Methyl-*meta*-difluor-AE besteht; und
wobei die dritte chemilumineszierende Verbindung ausgewählt wird aus der Gruppe, die aus üblichen Acridiniumester (AE), Naphthyl-AE, *ortho*-AE, 1- oder 3-Methyl-AE, 2-Methyl-AE, 2,7-Dimethyl-AE, 4,5-Dimethyl-AE, *ortho*-Dibrom-AE, *ortho*-Dimethyl-AE, *meta*-Dimethyl-AE, *ortho*-Methoxy-AE, *ortho*-Methoxy(cinnamyl)-AE, *ortho*-Methyl-AE, *ortho*-Fluor-AE, 1- oder 3-Methyl-*ortho*-fluor-AE und 1- oder 3-Methyl-*meta*-difluor-AE besteht,
mit der Massgabe, dass die ersten, zweiten und dritten chemilumineszierenden Verbindungen sich unterscheiden und voneinander unterscheidbar sind.

15. Das Verfahren nach Anspruch 14, wobei die erste chemilumineszierende Verbindung 1- oder 3-Methyl-*meta*-difluor-AE ist, die zweite chemilumineszierende Verbindung 1-oder 3-Methyl-AE ist und die dritte chemilumineszierende Verbindung *ortho*-Methoxy(cinnamyl)-AE ist.

16. Das Verfahren nach einem der Ansprüche 1 bis 15, wobei der Schritt zum In-Kontakt-Bringen einen Analyten beinhaltet, der eine Nukleinsäure, ein Protein, ein Lipid, ein Kohlenhydrat oder eine Verbindung, die eine Kombination davon ist, ist.

17. Das Verfahren nach einem der Ansprüche 1 bis 16, wobei der Schritt zum In-Kontakt-Bringen einen Analyten beinhaltet, der eine DNA oder RNA ist.

18. Das Verfahren nach einem der Ansprüche 1 bis 17, wobei der Schritt zum Bereitstellen ein Sondenreagenz verwendet, wobei mindestens eine markierte Sonde eine Nukleinsäure, ein Protein, ein Lipid, ein Kohlenhydrat oder eine Verbindung, die eine Kombination davon ist, ist.

19. Das Verfahren nach einem der Ansprüche 1 bis 18, wobei der Schritt zum Bereitstellen ein Sondenreagenz verwendet, wobei mindestens eine markierte Sonde eine DNA, eine RNA, ein DNA-Analogon, ein RNA-Analogon oder eine Kombination davon umfasst.

20. Ein Sondenreagenz zur Verwendung in dem Verfahren nach einem der Ansprüche 1 bis 19, das mindestens zwei unterschiedlich markierte Sonden umfasst, wobei jede markierte Sonde einen Target-bindenden Rest und einen detektierbaren Marker umfasst,
wobei der Target-bindende Rest jeder markierten Sonde spezifisch mit einem Targetbereich bindet, der sich von dem durch eine andere markierte Sonde im Sondenreagenz gebundenen Targetbereich unterscheidet, und
wobei jede markierte Sonde in einer Menge vorhanden ist, um einen Bereich von Analytkonzentrationen zu detektieren, der sich von dem durch eine andere Sonde im Reagenz detektierbaren Bereich von Analytkonzentrationen unterscheidet, und
wobei das Sondenreagenz eine erste Sonde enthält, die mindestens in einem 10-fachen molaren Überschuss relativ zur zweiten Sonde vorhanden ist, wodurch es dem Sondenreagenz ermöglicht wird, den Analyten über einen erweiterten Bereich von Analytkonzentrationen, der größer ist als der Bereich von Analytkonzentrationen, der durch eine einzelne Sonde im Sondenreagenz detektierbar ist, zu detektieren.

21. Das Sondenreagenz nach Anspruch 20, wobei das Sondenreagenz eine erste markierte Sonde beinhaltet, die in einer Menge vorhanden ist, die sich durch mindestens einen 100-fachen molaren Überschuss oder einen mindestens 1.000-fachen molaren Überschuss oder mindestens einen 10.000-fachen molaren Überschuss relativ zu einer zweiten im Sondenreagenz vorhandenen markierten Sonde unterscheidet.

22. Das Sondenreagenz nach Anspruch 20 oder 21, wobei jede markierte Sonde von jeder anderen markierten Sonde im Sondenreagenz unterscheidbar ist aufgrund:
unterschiedlicher spezifischer Aktivitäten der unterschiedlich markierten Sonden,
eines Signals, das eine Folge von getrennt unterscheidbaren Markern auf jeder der markierten Sonden ist,
oder einer Kombination davon.

23. Das Sondenreagenz nach einem der Ansprüche 20 bis 22, wobei jede markierte Sonde verglichen mit einer anderen markierten Sonde im Sondenreagenz eine andere spezifische Aktivität des Markers aufweist.

24. Das Sondenreagenz nach Anspruch 23, wobei eine markierte Sonde mit einer relativ hohen spezifischen Aktivität in einer molaren Menge vorhanden ist, die niedriger.ist als die molare Menge einer markierten Sonde mit einer relativ niedrigen spezifischen Aktivität im Sondenreagenz.

25. Das Sondenreagenz nach einem der Ansprüche 20 bis 24, wobei jede markierte Sonde ein Nukleinsäureoligonukleotid mit einem Target-bindenden Rest, der eine Basensequenz ist, ist.

26. Das Sondenreagenz nach einem der Ansprüche 20 bis 25, wobei jede markierte Sonde einen getrennt voneinander detektierbaren Marker aufweist, der ein Acridiniumesterderivat ist.

## Revendications

1. Méthode de détection d'une substance à analyser sur une plage de concentrations dans un seul échantillon, comprenant les étapes de :
fourniture d'un réactif à sondes comprenant au moins deux sondes différentes marquées,
dans laquelle chaque sonde marquée comprend un fragment de liaison cible et un marqueur détectable,
dans laquelle le fragment de liaison cible de chaque sonde marquée se lie spécifiquement à une région cible qui diffère de la région cible liée par une autre sonde marquée dans le réactif à sondes, et
dans laquelle chaque sonde marquée est présente dans une quantité pouvant détecter une plage de concentrations de la substance à analyser qui diffère de la plage de concentrations de la substance à analyser détectable par une autre sonde dans le réactif, permettant ainsi au réactif à sondes de détecter une plage étendue de concentrations de la substance à analyser qui est supérieure à la plage de concentrations de la substance à analyser détectable par une seule sonde marquée dans le réactif à sondes ;
mise en contact dans un seul récipient du réactif à sondes avec un échantillon contenant la substance à analyser,
dans laquelle le réactif à sondes contient une première sonde en excès molaire d'au moins 10 fois relativement à une deuxième sonde ;
incubation de l'échantillon et du réactif à sondes dans des conditions favorisant la liaison spécifique du fragment de liaison cible de chaque sonde marquée à la région cible de la substance à analyser, produisant ainsi un complexe de liaison spécifique comprenant la substance à analyser et au moins une sonde marquée ; et
détection de la présence du complexe de liaison spécifique par détection d'un signal émis par le marqueur indiquant la présence de ladite au moins une sonde dans le complexe de liaison spécifique, indiquant ainsi la présence de la substance à analyser dans l'échantillon à l'intérieur de la plage de concentrations de la substance à analyser détectable par la sonde dans le complexe de liaison spécifique.

2. Méthode selon la revendication 1, dans laquelle les étapes de mise en contact et d'incubation sont effectuées dans un seul récipient.

3. Méthode selon la revendication 1 ou la revendication 2, dans laquelle l'étape de détection mesure un signal émis par le marqueur détectable qui est caractéristique de la cinétique d'une réaction.

4. Méthode selon l'une quelconque des revendications 1 à 3, dans laquelle l'étape de fourniture utilise un réactif à sondes dans lequel l'activité spécifique d'une sonde marquée diffère de l'activité spécifique d'une autre sonde marquée dans le réactif à sondes.

5. Méthode selon l'une quelconque des revendications 1 à 4, dans laquelle l'étape de fourniture utilise un réactif à sondes dans lequel au moins une sonde marquée porte un marqueur détectable qui est un ligand pouvant se lier spécifiquement à un partenaire de liaison produisant un signal.

6. Méthode selon l'une quelconque des revendications 1 à 4, dans laquelle l'étape de fourniture utilise un réactif à sondes dans lequel au moins une sonde marquée porte un marqueur détectable qui est un marqueur direct, et l'étape de détection mesure un signal émanant du marqueur direct.

7. Méthode selon l'une quelconque des revendications 1 à 4, dans laquelle l'étape de fourniture utilise un réactif à sondes dans lequel au moins une sonde marquée porte un marqueur détectable qui est un marqueur fluorescent, un marqueur luminescent, un marqueur radioactif, un chromophore, un enzyme, ou un substrat enzymatique.

8. Méthode selon l'une quelconque des revendications 1 à 6, dans laquelle l'étape de fourniture utilise un réactif à sondes dans lequel au moins une sonde marquée porte un marqueur détectable qui est un composé chimiluminescent.

9. Méthode selon l'une quelconque des revendications 1 à 8, dans laquelle l'étape de mise en contact utilise le réactif à sondes dans lequel la première sonde marquée est en excès molaire d'au moins 100 fois, 1 000 fois ou 10 000 fois relativement à la deuxième sonde marquée.

10. Méthode selon l'une quelconque des revendications 1 à 9, dans laquelle le réactif à sondes comprend la première sonde marquée ayant un marqueur détectable qui est un premier composé chimiluminescent et la deuxième sonde marquée ayant un marqueur détectable qui est un deuxième composé chimiluminescent, dans laquelle le premier composé chimiluminescent et le deuxième composé chimiluminescent sont des marqueurs pouvant être individuellement distingués.

11. Méthode selon la revendications 10, dans laquelle le premier composé chimiluminescent est choisi dans le groupe constitué d'ester d'acridine standard (AE), naphthyl-AE, ortho-AE, 1- ou 3-méthyl-AE, 2-méthyl-AE, 2,7-diméthyl-AE, 4,5-diméthyl-AE, ortho-dibromo-AE, ortho-diméthyl-AE, méta-diméthyl-AE, ortho-méthoxy-AE, ortho-méthoxy(cinnamyl)-AE, ortho-méthyl-AE, ortho-fluoro-AE, 1-ou 3-méthyl-ortho-fluoro-AE et 1- ou 3-méthyl-méta-difluoro-AE ; et
dans laquelle le deuxième composé chimiluminescent est choisi dans le groupe constitué d'ester d'acridine standard (AE), naphthyl-AE, ortho-AE, 1-ou 3-méthyl-AE, 2-méthyl-AE, 2,7-diméthyl-AE, 4,5-diméthyl-AE, ortho-dibromo-AE, ortho-diméthyl-AE, méta-diméthyl-AE, ortho-méthoxy-AE, ortho-méthoxy(cinnamyl)-AE, ortho-méthyl-AE, ortho-fluoro-AE, 1- ou 3-méthyl-ortho-fluoro-AE et 1- ou 3-méthyl-méta-difluoro-AE,
à condition que le premier composé chimiluminescent soit différent et se distingue du deuxième composé chimiluminescent.

12. Méthode selon la revendication 11, dans laquelle le premier composé chimiluminescent est ortho-fluoro-AE, et le deuxième composé chimiluminescent est 2-méthyl-AE.

13. Méthode selon la revendication 10, dans laquelle le réactif à sondes comprend en outre une troisième sonde marquée ayant un marqueur pouvant être individuellement distingué qui est un troisième composé chimiluminescent.

14. Méthode selon la revendication 13, dans laquelle le premier composé chimiluminescent est choisi dans le groupe constitué d'ester d'acridine standard (AE), naphthyl-AE, ortho-AE, 1- ou 3-méthyl-AE, 2-méthyl-AE, 2,7-diméthyl-AE, 4,5-diméthyl-AE, ortho-dibromo-AE, ortho-diméthyl-AE, méta-diméthyl-AE, ortho-méthoxy-AE, ortho-méthoxy(cinnamyl)-AE, ortho-méthyl-AE, ortho-fluoro-AE, 1-ou 3-méthyl-ortho-fluoro-AE et 1- ou 3-méthyl-méta-difluoro-AE ;
dans laquelle le deuxième composé chimiluminescent est choisi dans le groupe constitué d'ester d'acridine standard (AE), naphthyl-AE, ortho-AE, 1-ou 3-méthyl-AE, 2-methyl-AE, 2,7-diméthyl-AE, 4,5-diméthyl-AE, ortho-dibromo-AE, ortho-diméthyl-AE, méta-diméthyl-AE, ortho-méthoxy-AE, ortho-méthoxy(cinnamyl)-AE, ortho-méthyl-AE, ortho-fluoro-AE, 1- ou 3-méthyl-ortho-fluoro-AE et 1- ou 3-méthyl-méta-difluoro-AE ; et
dans laquelle le troisième composé chimiluminescent est choisi dans le groupe constitué d'ester d'acridine standard (AE), naphthyl-AE, ortho-AE, 1-ou 3-méthyl-AE, 2-méthyl-AE, 2,7-diméthyl-AE, 4,5-diméthyl-AE, ortho-dibromo-AE, ortho-diméthyl-AE, méta-diméthyl-AE, ortho-méthoxy-AE, ortho-méthoxy(cinnamyl)-AE, ortho-méthyl-AE, ortho-fluoro-AE, 1- ou 3-méthyl-ortho-fluoro-AE et 1- or 3-méthyl-méta-difluoro-AE,
à condition que le premier, le deuxième et le troisième composés chimiluminescents diffèrent et puissent être distingués les uns des autres.

15. Méthode selon la revendication 14, dans laquelle le premier composé chimiluminescent est 1- ou 3-méthyl-méta-difluoro-AE, le deuxième composé chimiluminescent est 1- ou 3-méthyl-AE, et le troisième composé chimiluminescent est ortho-méthoxy(cinnamyl)-AE.

16. Méthode selon l'une quelconque des revendications 1 à 15, dans laquelle l'étape de mise en contact inclut une substance à analyser qui est un acide nucléique, une protéine, un lipide, un glucide ou un composé qui est une combinaison de ceux-ci.

17. Méthode selon l'une quelconque des revendications 1 à 16, dans laquelle l'étape de mise en contact inclut une substance à analyser qui est un ADN ou un ARN.

18. Méthode selon l'une quelconque des revendications 1 à 17, dans laquelle l'étape de fourniture utilise un réactif à sondes dans lequel au moins une sonde marquée est un acide nucléique, une protéine, un lipide, un glucide ou un composé qui est une combinaison de ceux-ci.

19. Méthode selon l'une quelconque des revendications 1 à 18, dans laquelle l'étape de fourniture utilise un réactif à sondes dans lequel au moins une sonde marquée comprend un ADN, un ARN, un analogue d'ADN, un analogue d'ARN, ou une combinaison de ceux-ci.

20. Réactif à sondes à utiliser dans la méthode selon l'une quelconque des revendications 1 à 19, comprenant au moins deux sondes différentes marquées, chaque sonde marquée comprenant un fragment de liaison cible et un marqueur détectable,
dans lequel le fragment de liaison cible de chaque sonde marquée se lie spécifiquement à une région cible qui diffère de la région cible liée par une autre sonde marquée dans le réactif à sondes, et
dans lequel chaque sonde marquée est présente dans une quantité pouvant détecter une plage de concentrations de la substance à analyser qui diffère d'une plage de concentrations de la substance à analyser détectable par une autre sonde dans le réactif, et
dans lequel le réactif à sondes contient une première sonde en excès molaire d'au moins 10 fois relativement à une deuxième sonde, permettant ainsi au réactif à sondes de détecter la substance à analyser sur une plage étendue de concentrations de la substance à analyser qui est supérieure à la plage de concentrations de la substance à analyser détectable par une seule sonde dans le réactif à sondes.

21. Réactif à sondes selon la revendication 20, dans lequel le réactif à sondes contient une première sonde marquée présente dans une quantité qui diffère par un excès molaire d'au moins 100 fois, ou par un excès molaire d'au moins 1 000 fois, ou par un excès molaire d'au moins 10 000 fois relativement à une deuxième sonde marquée présente dans le réactif à sondes.

22. Réactif à sondes selon la revendication 20 ou la revendication 21, dans lequel chaque sonde marquée se distingue des autres sondes marquées dans le réactif à sondes en raison :
des activités spécifiques différentes des différentes sondes marquées,
d'un signal émanant des marqueurs pouvant être individuellement distingués sur chacune des sondes marquées,
ou d'une combinaison de ceux-ci.

23. Réactif à sondes selon l'une quelconque des revendications 20 à 22, dans lequel chaque sonde marquée a une activité spécifique du marqueur différente comparée à une autre sonde marquée dans le réactif à sondes.

24. Réactif à sondes selon la revendication 23, dans lequel une sonde marquée ayant une activité spécifique relativement forte est présente dans une quantité molaire qui est inférieure à une quantité molaire d'une sonde marquée ayant une activité spécifique relativement faible dans le réactif à sondes.

25. Réactif à sondes selon l'une quelconque des revendications 20 à 24, dans lequel chaque sonde marquée est un oligonucléotide d'acides nucléiques ayant un fragment de liaison cible qui est une séquence de bases.

26. Réactif à sondes selon l'une quelconque des revendications 20 à 25, dans lequel chaque sonde marquée porte un marqueur individuellement détectable qui est un dérivé d'ester d'acridine.
